# EUROPEAN PATENT APPLICATION

(11) **EP 3 563 815 A1**
(43) Date of publication of application: **06.11.2019**
(21) Application number: 17887918.5
(22) Date of filing: 25.12.2017
(51) Int. Cl.: A61F 9/007

(54) **SURGICAL TOOL**

(30) Priority: 27.12.2016 JP 2016253671
(71) Applicant: Rohto Pharmaceutical Co., Ltd., Osaka-shi Osaka 544-8666 (JP)
(72) Inventor: YABE, Tsutomu, Osaka-shi Osaka 544-8666 (JP); ISHII, Atsuo, Osaka-shi Osaka 544-8666 (JP)
(74) Representative: Jacob, Reuben Ellis
(86) International application number: PCT/JP2017/046437
(87) International publication number: WO 2018/123973

(57) **Abstract**

Provided is a surgical tool for ophthalmology that includes a nozzle portion capable of sucking a liquid, in which the nozzle portion includes an inner tube which is provided with an inner through-hole allowing the liquid to pass therethrough and an outer tube which accommodates the inner tube and is provided with an outer through-hole allowing the liquid to pass therethrough, in which the outer tube and the inner tube are disposed to be relatively movable, and in which the outer through-hole and the inner through-hole are disposed so that an overlapping region is changeable by the relative movement of the outer tube and the inner tube.

## Description

### Technical Field

The invention relates to a surgical tool for ophthalmology.

### Background Art

A handpiece used in cataract surgery (lens reconstruction technique) is known (see Patent Literature 1). In the lens reconstruction technique, for example, a nozzle distal end of an ultrasonic handpiece is inserted into an atrioventricular chamber of an eyeball to crush and suck lens nucleus. In this case, a viscoelastic substance is injected into the atrioventricular chamber to maintain a shape. Thereafter the nozzle of the ultrasonic handpiece is replaced with a nozzle of a perfusion handpiece, then cortex or cortex and lens nucleus not removed by the ultrasonic handpiece are sucked out while being washed by perfusion.

### Citation List

### Patent Literature

Patent Literature 1: Japanese Unexamined Patent Publication No. 2013-180004

### Summary of Invention

### Technical Problem

For example, there is a possibility that viscosity changes depending on a remaining amount of the viscoelastic substance injected into the atrioventricular chamber or a preferred suction amount changes depending on a remaining amount of cortex during perfusion suction, but in general, nozzle replacement which is performed by inserting and extracting a nozzle into and from the atrioventricular chamber is not frequently performed in consideration of a burden of a patient Meanwhile, since the nozzle replacement is not performed, it is difficult to adjust a suction amount depending on a situation. Since a time necessary for perfusion or the like increases, there is a possibility that a patient feels burdensome.

An object of an aspect of the invention is to provide a surgical tool for ophthalmology capable of adjusting a suction amount depending on a situation during ophthalmic surgery.

### Solution to Problem

An aspect of the invention is a surgical tool for ophthalmology that includes a nozzle portion capable of sucking a liquid, in which the nozzle portion includes an inner tube which is provided with an inner through-hole allowing the liquid to pass therethrough and an outer tube which accommodates the inner tube and is provided with an outer through-hole allowing the liquid to pass therethrough, in which the outer tube and the inner tube are disposed to be relatively movable, and in which the outer through-hole and the inner through-hole are disposed so that an overlapping region is changeable by the relative movement of the outer tube and the inner tube.

In the surgical tool, it is possible to change the overlapping region between the outer through-hole and the inner through-hole by relatively moving the outer tube and the inner tube. When the overlapping region is increased, the suction amount increases. Meanwhile, when the overlapping region is decreased, the suction amount decreases. That is, it is possible to adjust a suction amount depending on a situation without requiring an excessive burden of a patient by adjusting the overlapping region.

In the surgical tool, the outer through-hole and the inner through-hole are disposed so that at least a part of one of the through-holes overlaps a movement locus of at least the other one that moves. In this aspect, since it is possible to reliably form an overlapping region with respect to at least a part of the one by moving the other one and to easily change the overlapping region, the adjustment is easy.

In the surgical tool, the inner tube may be movable with respect to the outer tube and at least a part of the outer through-hole may be disposed to overlap the movement locus of the inner through-hole. Since it is possible to adjust the suction amount just by moving the inner tube without moving the outer tube, it is possible to further reduce a burden of a patient.

In the surgical tool, the outer through-hole may be provided in a side portion of the outer tube and the inner through-hole may be provided in a side portion of the inner tube. It is possible to effectively suck a liquid from the side portion instead of the distal end sides of the outer tube and the inner tube and to adjust the suction amount.

In the surgical tool, the outer tube and the inner tube may have a common axis and the surgical tool may further include a holding portion which holds the outer tube and the inner tube to be relatively movable in a rotation direction with respect to the axis. When the suction amount can be adjusted by the relative movement in the rotation direction instead of the axial direction, a design burden in consideration of the movement amount in the axial direction is not necessary and hence a compact size in the axial direction is advantageous.

In the surgical tool, the outer through-hole may be larger than the inner through-hole and the inner through-hole may be housed in the outer through-hole in a state in which the outer through-hole and the inner through-hole completely overlap each other. The maximum suction amount is dependent on the diameter of the inner through-hole, but since the inner through-hole is housed in the outer through-hole in an overlapping state, a desired maximum suction amount corresponding to the diameter of the inner through-hole can be appropriately obtained without any disturbance of the outer tube.

In the surgical tool, the inner tube may be provided with a plurality of the inner through-holes having different sizes. Since the plurality of inner through-holes having different sizes are provided in the inner tube, the suction amount can be easily adjusted gradually and hence the operability is improved.

The surgical tool may further include: a movable body which is fixed to the inner tube and is provided with an inner passage communicating with the inner tube; and a casing portion which is fixed to the outer tube, accommodates the movable body, and rotatably supports the movable body, in which the movable body may be provided with a handle portion which protrudes in a direction orthogonal to the axis and penetrates the casing portion to be exposed from the casing portion. Since it is possible to simply adjust the suction amount by rotating the handle portion, it is possible to improve operability.

Further, according to an aspect of the invention, provided is a surgical tool for ophthalmology including: a nozzle portion which is able to suck a liquid; and a holding portion which is fixed to the nozzle portion, in which the nozzle portion includes an inner tube through which a liquid passes and which is provided with a plurality of inner through-holes having different sizes and an outer tube which is provided with an outer through-hole allowing the liquid to flow therethrough, accommodates the inner tube, and has a common axis to the inner tube, in which the holding portion includes a movable body which is fixed to the inner tube and is provided with an inner passage communicating with the inner tube and a casing portion which is fixed to the outer tube, accommodates the movable body, and rotatably supports the movable body, and in which at least a part of the outer through-hole is disposed to overlap the movement locus of the plurality of inner through-holes.

In the surgical tool, at least a part of the outer through-hole can overlap any inner through-hole having a different size as the inner tube rotates with respect to the outer tube. For example, in an embodiment in which two inner through-holes having different sizes are provided, the liquid suction amount increases when the outer through-hole overlaps the large inner through-hole and the liquid suction amount decreases when the outer through-hole overlaps the small inner through-hole. That is, it is possible to adjust a suction amount depending on a situation without requiring an excessive burden of a patient by selecting the inner through-hole to overlap the outer through-hole.

In the surgical tool, the inner passage may include an enlarged passage portion which has an inner diameter larger than that of the inner tube and a length in a direction along the axis of the enlarged passage portion may be longer than a length of the inner tube. Since the inner diameter of the inner tube is smaller than the inner diameter of the enlarged passage portion, a resistance when the liquid passes through the inner tube becomes larger than a resistance when the liquid passes through the enlarged passage portion. However, since the length of the enlarged passage portion in the axial direction is longer than the length of the inner tube, it is possible to decrease the resistance in all of the inner tube and the inner passage and to easily increase the flow amount of the liquid passing through the inner through-hole.

In the surgical tool, the plurality of inner through-holes may be a first inner through-hole and a second inner through-hole larger than the first inner through-hole, the movable body may be provided with a handle portion which protrudes in a direction orthogonal to the axis and penetrates the casing portion to be exposed from the casing portion, the handle portion may be provided with a guide hole which penetrates in a direction along the axis and is elongated along a rotation direction around the axis when viewed from a direction along the axis, the casing portion may include a guide rod which is inserted through the guide hole and is relatively movable inside the guide hole, the guide hole may be formed by a first guide surface which extends along the rotation direction around the axis, a second guide surface which is away from the axis in relation to the first guide surface and extends to face the first guide surface, and a pair of stopper surfaces which is provided at both ends of the first guide surface and the second guide surface and comes into contact with the guide rod to regulate the relative movement of the guide rod, the first inner through-hole and the outer through-hole may overlap each other at a position in which the guide rod comes into contact with one stopper surface of the pair of stopper surfaces, and the second inner through-hole and the outer through-hole may overlap each other at a position in which the guide rod comes into contact with the other stopper surface.

When the guide rod comes into contact with one stopper surface, the first inner through-hole and the outer through-hole are aligned to overlap each other. Then, when the guide rod comes into contact with the other stopper surface, the second inner through-hole and the outer through-hole are aligned to overlap each other. As a result, since it is easy to align the first inner through-hole and the outer through-hole and to align the second inner through-hole and the outer through-hole, operability is improved.

In the surgical tool, the first inner through-hole and the second inner through-hole may be provided at a position in which a rotation angle around the axis is 180°. That is, the first inner through-hole and the second inner through-hole are provided at a position facing each other around the axis. When the first inner through-hole and the second inner through-hole face each other, it is easy to increase the size and to increase the flow amount of the liquid passing through the first inner through-hole and the second inner through-hole.

In the surgical tool, the casing portion and the movable body may be separable from each other, the casing portion may include a first casing to which the outer tube is fixed and a second casing which is opposite to the first casing with the handle portion interposed therebetween, and the guide rod may include a rod main body which is fixed to the first casing and a head portion which is attached to an end of the rod main body and is separably attached to the second casing. The casing portion can be separated into the first casing and the second casing.

In the surgical tool, the rod main body may have a linear shape extending along the axis, an outer diameter of the head portion may be larger than a maximum outer diameter of the rod main body, the guide hole may include a first region in which a distance between the first guide surface and the second guide surface is larger than the maximum outer diameter of the rod main body and smaller than the outer diameter of the head portion and a second region in which a distance between the first guide surface and the second guide surface is larger than the outer diameter of the head portion, and the second region may be narrower than the first region in a rotation direction around the axis and extend along the axis. In a case in which the first casing and the second casing are separated from each other so that the guide rod is extracted from the guide hole, the head portion of the guide rod can pass through the second region, but cannot pass through the first region. Further, since the second region extends along the axis, it is possible to extract the guide rod in a direction along the axis. As a result, it is possible to separate the first casing in a direction along the axis with respect to the movable body.

In the surgical tool, the movable body may include a protrusion shaft body which protrudes from the handle portion and is separably accommodated in the first casing along the axis and an annular seal member that is attached to the protrusion shaft body and is in contact with the first casing, the first casing may include a concave portion which accommodates the protrusion shaft body and is in contact with the seal member, the rod main body may include a remaining portion which forms a gap between the handle portion and the head portion, and a distance from the seal member to an end portion near the handle portion of the concave portion may be equal to or longer than a length in a direction along the axis of the remaining portion.

When the movement of the first casing deviates from a direction along the axis at the time of separating the first casing from the movable body, there is a possibility that the inner tube may be damaged due to the interference between the inner tube and the outer tube. This deviation easily occurs in moments when a load applied to the seal member is released due to the seal member separating from the concave portion of the first casing. In the surgical tool, when only the first casing is moved by the length of the remaining portion of the rod main body with respect to the movable body, the head portion of the guide rod reaches the guide hole. In accordance with this movement, the seal member on the side of the movable body moves in the opposite direction. Here, the distance from the seal member to the end portion near the handle portion of the concave portion is equal to or longer than the length of the remaining portion. That is, the seal member is not separated from the concave portion at a timing in which the head portion reaches the guide hole. Next, when the reaching position is within the first region even when the head portion reaches the guide hole, the head portion cannot pass through the guide hole and hence the movement of the movable body is regulated. Thus, in order to further move the movable body, there is a need to adjust the position so that the head portion reaches the second region by rotating the movable body. Thus, in the surgical tool, there is a possibility that an operator has a chance of adjusting the position of the head portion before the seal member is separated from the concave portion. Accordingly, it is easy to suppress the damage of the inner tube by prompting a careful movement operation of the first casing or the like.

In the surgical tool, a distance from the seal member to an end portion near the handle portion of the concave portion may be the same as a length in a direction along the axis of the remaining portion. In the surgical tool, there is a possibility that the operator can adjust the position of the head portion immediately before the seal member is separated from the concave portion. Accordingly, it is easy to suppress the damage of the inner tube by more appropriately prompting a careful movement operation of the first casing or the like.

### Advantageous Effects of Invention

According to the invention, it is possible to adjust a suction amount depending on a situation without requiring an excessive burden of a patient during ophthalmic surgery.

### Brief Description of Drawings

FIG 1 is a diagram according to one embodiment, where FIG. 1(a) is a front view illustrating a state in which a sleeve is attached to an I/A handpiece and FIG. 1(b) is a front view illustrating a state in which the sleeve is separated from the I/A handpiece.
FIG. 2 is a front view illustrating a state in which the I/A handpiece is disassembled.
FIG. 3 is an enlarged cross-sectional view illustrating a grip portion of the I/A handpiece.
FIG. 4 is a cross-sectional view taken along a line IV-IV of FIG. 3.
FIG. 5 is an enlarged cross-sectional view illustrating a nozzle portion of the I/A handpiece.
FIG. 6 is a diagram illustrating an outer through-hole, where FIG. 6(a) is a front view and FIG. 6(b) is a cross-sectional view taken aloneg a line b-b of FIG. 6(a).
FIG 7 is a diagram illustrating an inner through-hole, where FIG. 7(a) is a front view illustrating a first inner through-hole and FIG. 7(b) is a front view illustrating a second inner through-hole.
FIG. 8 is a cross-sectional view of an inner tube, where FIG 8(a) is a cross-sectional view cut along a plane which is orthogonal to the axis of the inner tube and passes through the centers of the first inner through-hole and the second inner through-hole, FIG. 8(b) is a cross-sectional view taken along a line b-b of FIG. 8(a), and FIG. 8(c) is a cross-sectional view taken along a line c-c of FIG 8(a).
FIG 9 is an enlarged view illustrating the vicinity of a distal end of the nozzle portion, where FIG. 9(a) is a front view illustrating a state in which the first inner through-hole and the outer through-hole overlap each other and FIG. 9(b) is a front view illustrating a state in which the second inner through-hole and the outer through-hole overlap each other.
FIG. 10 is a diagram illustrating a state in which a perfusion liquid containing cortex is sucked during cataract surgery, where FIG. 10(a) is a perspective view and FIG. 10(b) is a schematic plan view.
FIG. 11 is a front view illustrating the I/A handpiece according to one embodiment.
FIG. 12 is a left view illustrating the I/A handpiece according to one embodiment.
FIG 13 is a right view illustrating the I/A handpiece according to one embodiment.
FIG. 14 is a rear view illustrating the I/A handpiece according to one embodiment.
FIG. 15 is a bottom view illustrating the I/A handpiece according to one embodiment.
FIG. 16 is a plan (top) view illustrating the I/A handpiece according to one embodiment.
FIG 17 is a schematic diagram illustrating an I/A handpiece according to a second embodiment, where FIG. 17(a) is a schematic cross-sectional view illustrating a state in which an acute angle portion of an inner tube overlaps an outer through-hole and FIG 17(b) is a schematic cross-sectional view illustrating a state in which an obtuse angle portion of the inner tube overlaps the outer through-hole.
FIG. 18 is a schematic diagram illustrating an I/A handpiece according to a third embodiment, where FIG 18(a) is a schematic end view and a cross-sectional view illustrating a state in which an overlapping region between an inner through-hole and an outer through-hole is not formed, FIG. 18(b) is a schematic end view and a cross-sectional view illustrating a state in which a half overlapping region is formed, and FIG. 18(c) is a schematic end view and a cross-sectional view illustrating a state in which the inner through-hole is housed in the outer through-hole.
FIG 19 is a schematic diagram illustrating a nozzle portion of an I/A handpiece according to a fourth embodiment, where FIG. 19(a) is a schematic front view illustrating a state in which an overlapping region is not formed and FIG. 19(b) is a schematic front view illustrating a state in which an inner through-hole is housed in an outer through-hole.
FIG 20 is a diagram illustrating an I/A handpiece according to a fifth embodiment and is a cross-sectional view cut along a plane passing through an axis.
FIG. 21 is a cross-sectional view illustrating an outer tube and a discharge side casing of the I/A handpiece according to the fifth embodiment.
FIG 22 is a cross-sectional view illustrating a movable body and an inner tube of the I/A handpiece according to the fifth embodiment.
FIG. 23 is an enlarged view illustrating a connection portion between a casing portion and the movable body of the I/A handpiece according to the fifth embodiment, where FIG. 23(a) is a diagram illustrating a state in which the movable body and the casing portion are coupled to each other and FIG 23(b) is a diagram illustrating a state in which the casing portion and the movable body are being separated from each other.
FIG. 24 is an enlarged cross-sectional view illustrating a nozzle portion of the I/A handpiece according to the fifth embodiment.
FIG 25 is a cross-sectional view taken along a line XXV-XXV of FIG 24.
FIG. 26 is a cross-sectional view taken along a line XXVI-XXVI of FIG. 20.
FIG 27 is an enlarged view illustrating the vicinity of a distal end of the nozzle portion, where FIG. 27(a) is a front view illustrating a state in which a second inner through-hole and an outer through-hole overlap each other and FIG. 27(b) is a front view illustrating a state in which a first inner through-hole and the outer through-hole overlap each other.

### Description of Embodiments

Hereinafter, embodiments according to the invention will be described in detail with reference to the drawings. For the sake of convenience, substantially the same components are denoted by the same reference numerals and a description thereof may be omitted. Further, in the description below, a "distal end" or a "distal end side" is a concept which means a distal end of a nozzle portion to be described later or a side close to the distal end and a "base end" or a "base end side" is a concept which means an end portion opposite to the distal end of the nozzle portion or a side away from the distal end. An axis means each of an axis of an inner tube, an axis of an outer tube, or the like, but since all of these axes are common in the following embodiments, they are described as a common axis.

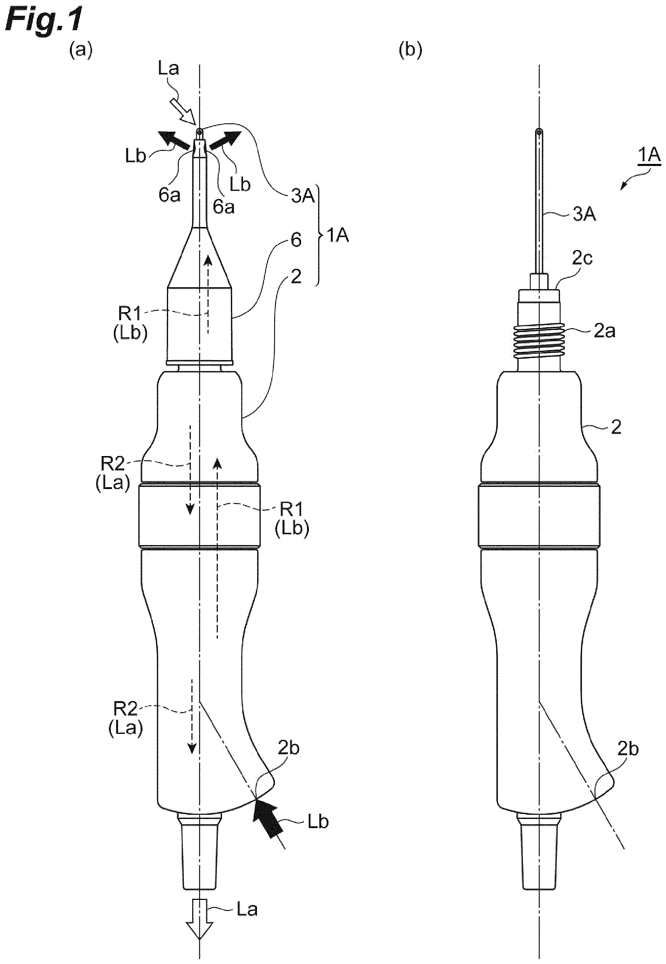

An I/A handpiece 1A is an example of a surgical tool for ophthalmology according to the embodiment and is generally called an ophthalmic perfusion/suction tube. As shown in FIG. 1(a), the I/A handpiece 1A includes a grip portion (a holding portion) 2 which is gripped by a practitioner, a nozzle portion 3A which is able to suck a liquid (a suction liquid) La, and a sleeve 6 which guides to the distal end side of the nozzle portion 3A a liquid (a perfusion liquid) Lb fed into the I/A handpiece 1A and discharged through the grip portion 2. The suction liquid La is an example of a liquid sucked by the nozzle portion 3A.

The nozzle portion 3A is a double tube and is attached to a distal end side of the grip portion 2. The grip portion 2 is provided with a supply path R1 which, receives the perfusion liquid Lb and sends the liquid to the distal end side and a discharge path R2 which sends the suction liquid La sucked through the nozzle portion 3A to a base end side. The sleeve 6 covers the distal end of the grip portion 2 while passing the nozzle portion 3A at the inside and is screwed into a screw portion 2a provided in the grip portion 2. A distal end of the sleeve 6 is opened and a distal end of the nozzle portion 3A is exposed. A discharge port 6a through which the perfusion liquid Lb is discharged is provided in the vicinity of the distal end of the sleeve 6.

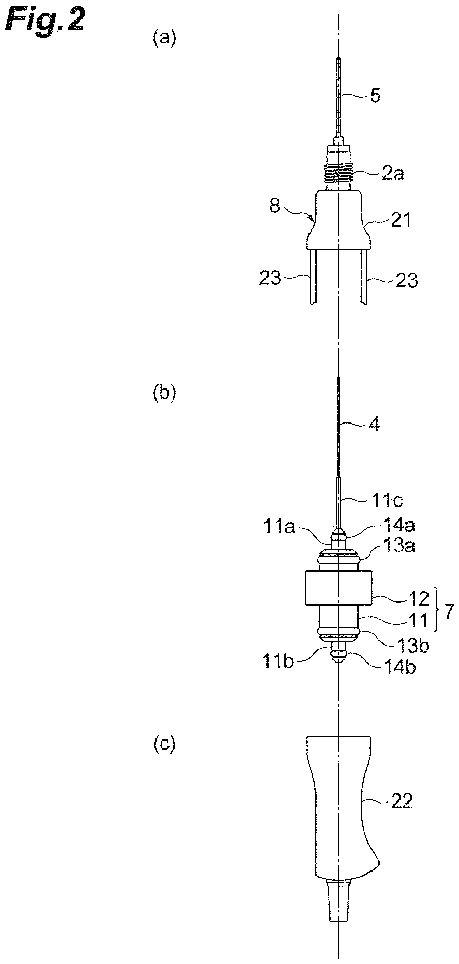

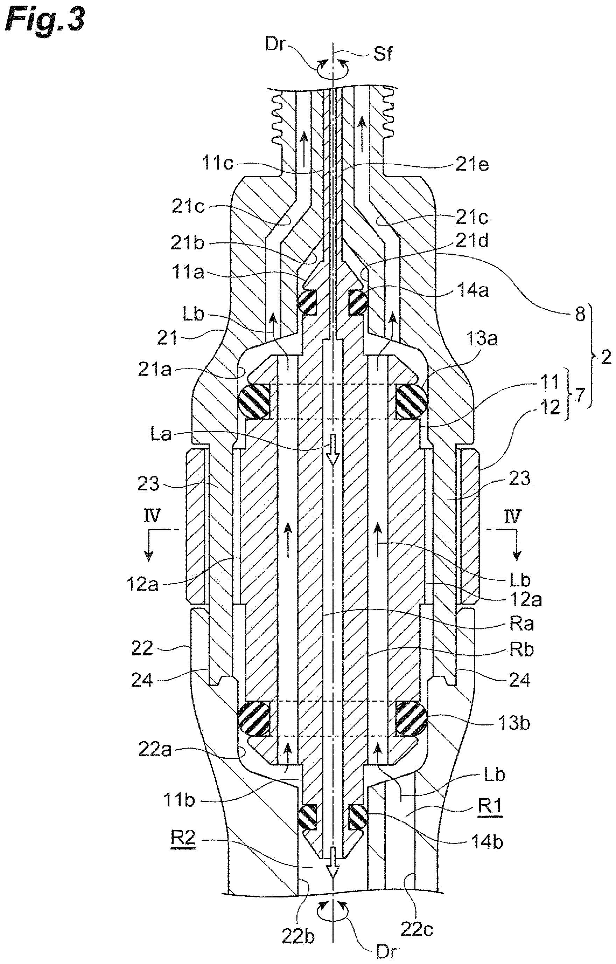

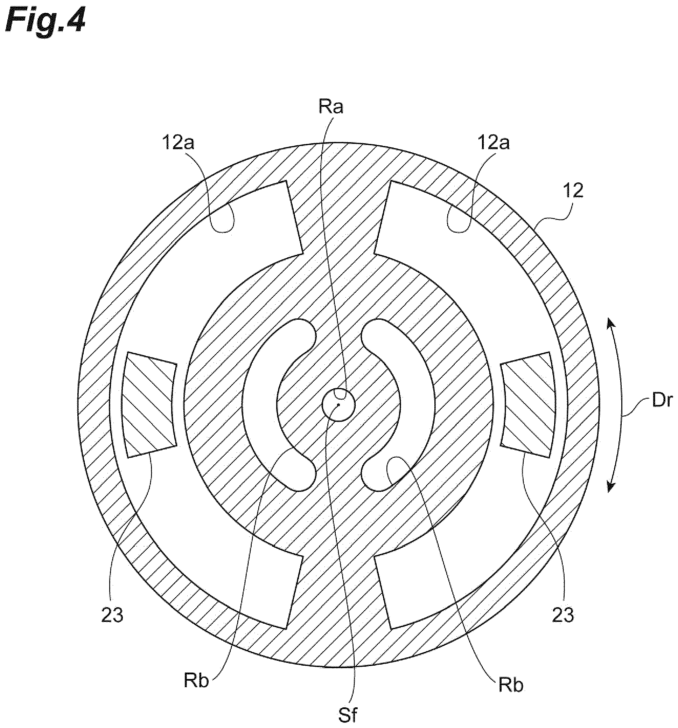

Hereinafter, a structure of each unit will be described in detail by focusing on a basic form in a state in which the sleeve 6 is separated with reference to FIGS. 1(b), 2, 3, and 4. The grip portion 2 includes a movable body 7 to which an inner tube 4 of the nozzle portion 3A is fixed and a casing portion 8 to which an outer tube 5 of the nozzle portion 3A is fixed and which accommodates the movable body 7 and supports the movable body 7 in a rotatable manner. The movable body 7 (see FIG. 3) is provided with an inner passage Ra which communicates with the inner tube 4 and an outer passage Rb through which the perfusion liquid Lb passes.

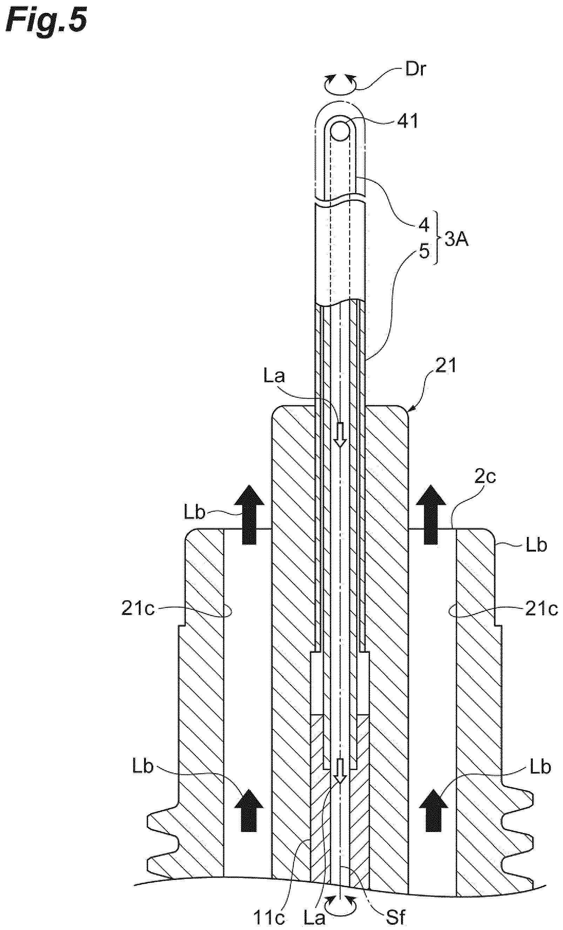

The movable body 7 includes a tubular body portion 11 and a handle portion 12 which extends in the radial direction from the body portion 11. The radial direction means a direction (a centrifugal direction) which is orthogonal to an axis Sf of the rotating movable body 7. The handle portion 12 according to the embodiment has a disk shape, but can have various shapes in consideration of the operability of the practitioner. O-rings (seal members) 13a and 13b are respectively attached to the distal end side and the base end side so that the handle portion 12 is interposed therebetween at the outer periphery of the body portion 11. Further, a distal end tube 11 a of a small diameter protrudes from the distal end side of the body portion 11 and a base end tube 11b of a small diameter protrudes from the base end side. O-rings (seal members) 14a and 14b are respectively attached to the outer peripheries of the distal end tube 11a and the base end tube 11b. Further, the distal end tube 11a is further provided with a distal end attachment tube 11c of a smaller diameter and the inner tube 4 of the nozzle portion 3A is fixed to the distal end attachment tube 11c so as to have the same axis Sf(see FIG. 5).

The casing portion 8 includes an introduction side casing 22 which receives the perfusion liquid Lb and is disposed at the base end side and a discharge side casing 21 which discharges the perfusion liquid Lb and is disposed at the distal end side. The introduction side casing 22 is provided with an introduction side concave portion 22a which accommodates a portion of the base end side of the body portion 11 and a suction path 22b which communicates with the center of the introduction side concave portion 22a. The introduction side concave portion 22a and the suction path 22b communicate with each other so as to have the same axis Sf with respect to the body portion 11 of the movable body 7. Further, an introduction path 22c is provided in the introduction side casing 22 so as to avoid the suction path 22b, and the introduction path 22c connects an introduction port 2b of the perfusion liquid Lb (see FIG. 1) and the introduction side concave portion 22a to communicate.

The body portion 11 is inserted into the introduction side concave portion 22a with a gap interposed therebetween and the gap is liquid-tightly blocked by the O-ring 13b attached to the outer periphery of the body portion 11. Further, the base end tube 11b of the body portion 11 is inserted into the suction path 22b and a gap between the suction path 22b and the base end tube 11b is liquid-tightly blocked by the O-ring 14b. As a result, a space which is liquid-tightly kept is formed between the introduction side concave portion 22a and the body portion 11 so that the perfusion liquid Lb passes therethrough.

The discharge side casing 21 is provided with a discharge side concave portion 21a which accommodates a part of the distal end side of the body portion 11 and an inner tube insertion hole 21b which communicates with the center of the discharge side concave portion 21a. The discharge side concave portion 21a and the inner tube insertion hole 21b communicate with each other so as to have the same axis Sf with respect to the body portion 11 of the movable body 7. Further, a discharge passage 21c is provided in the discharge side casing 21 so as to avoid the inner tube insertion hole 21b, and the discharge passage 21c connects a discharge port 2c of the perfusion liquid Lb (see FIGS. 1 and 5) and the discharge side concave portion 21a to communicate.

The inner tube insertion hole 21b includes an insertion hole portion 21d into which the distal end tube 11a is inserted and a communication hole portion 21e into which the distal end attachment tube 11c is inserted in a rotatable manner. The inner tube 4 which is fixed to the distal end attachment tube 11c protrudes from the communication hole portion 21e and is inserted into the outer tube 5 in a rotatable manner.

The body portion 11 is inserted into the discharge side concave portion 21a with a gap interposed therebetween and the gap is blocked by the O-ring 13a attached to the outer periphery of the body portion 11. Further, the distal end tube 11a of the body portion 11 is inserted into the inner tube insertion hole 21b and a gap between the inner tube insertion hole 21b and the distal end tube 11a is blocked by the O-ring 14a. That is, a space, which is liquid-tightly kept is formed between the discharge side concave portion 21a and the body portion 11 so that the perfusion liquid Lb passes, is formed by the O-ring 13a attached to the outer periphery of the body portion 11 and the O-ring 14a attached to the outer periphery of the distal end tube 11a.

The discharge side casing 21 and the introduction side casing 22 are disposed with a gap interposed therebetween through the connection portion 23 and are integrally coupled to each other by the fitting of the connection portion 23 and the connection groove 24. Specifically, the discharge side casing 21 is provided with two (a plurality of) connection portions 23 which protrude toward the base end side and have a columnar shape. The two connection portions 23 are disposed while facing each other so as to be symmetrical with respect to the axis Sf of the movable body 7. Meanwhile, the introduction side casing 22 is provided with two connection grooves 24 which are fitted and coupled to the connection portion 23. The discharge side casing 21 and the introduction side casing 22 which accommodate the body portion 11 of the movable body 7 are integrated while being coupled to each other by the fitting between the connection portion 23 and the connection groove 24 so that the casing portion 8 is formed. A distal end of the connection portion 23 fitted to the connection groove 24 is a head portion.

The handle portion 12 of the movable body 7 protrudes in a direction orthogonal to the axis Sf and is exposed while penetrating the gap between the discharge side casing 21 and the introduction side casing 22. Further, the handle portion 12 is provided with an escaping hole 12a for escaping the interference with the connection portion 23. The escaping hole 12a is provided at two (a plurality of) positions in response to the number of the connection portions 23. The escaping hole 12a may be formed along a region on the handle portion 12 interfering with the connection portion 23 when the movable body 7 is rotated with respect to the casing portion 8 and in the embodiment, two arc-shaped escaping holes 12a are provided to be symmetrical with the axis Sf interposed therebetween.

Since the handle portion 12 protrudes while penetrating the casing portion 8, the handle portion functions as an axial stopper which regulates the movement of the movable body 7 in the direction of the axis Sf. Further, when the movable body 7 is rotated too much, the connection portion 23 of the discharge side casing 21 interferes with the escaping hole 12a provided in the handle portion 12 to regulate the rotation. That is, the escaping hole 12a and the connection portion 23 function as a rotation stopper which regulates the rotation of the movable body 7.

Next, the nozzle portion 3A will be described with reference to FIGS. 5, 6, 7, 8, and 9. The nozzle portion 3A is a double tube and includes the inner tube 4 which is provided with inner through-holes 41 and 42 and the outer tube 5 which accommodates the inner tube 4 and is provided with an outer through-hole 51. As described above, in the embodiment, the inner tube 4 fixed to the movable body 7 is rotatable with respect to the outer tube 5 fixed to the casing portion 8. However, an embodiment may be employed in which the outer tube 5 and the inner tube 4 are relatively movable and the outer tube 5 is rotatable with respect to the inner tube 4.

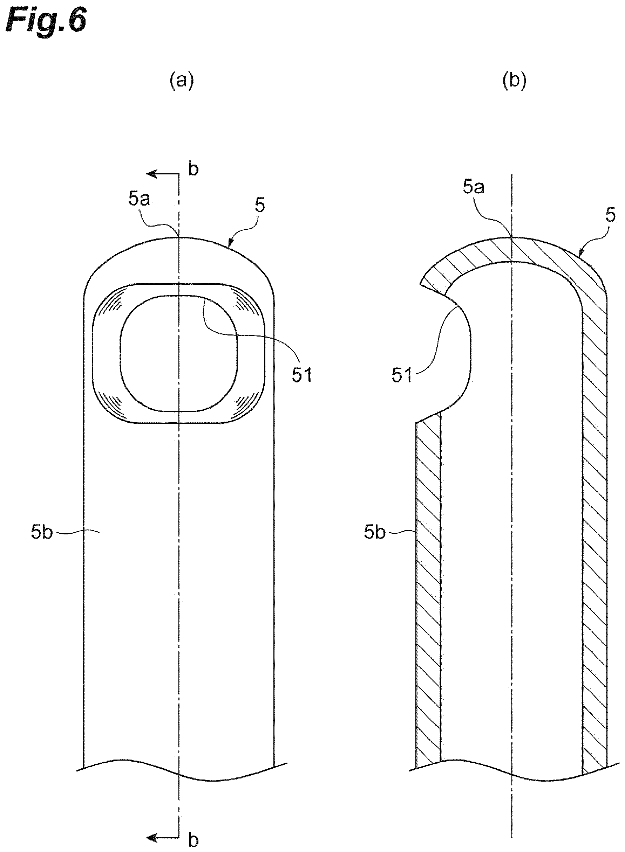

As shown in FIG. 6, the distal end 5a of the outer tube 5 is curved and sealed and the outer through-hole 51 is provided in a side portion 5b in the vicinity of the distal end 5a of the outer tube 5. The outer through-hole 51 penetrates the outer tube 5 to enable the inside and the outside to communicate. By the driving of a suction device (not shown) such as a venturi pump connected to the I/A handpiece 1A, for example, the suction liquid La containing cortex inside an atrioventricular chamber B passes. Furthermore, as will be described later, the distal end of the outer tube 5 may be opened in consideration of design and manufacturing convenience and superiority as long as the distal end of the inner tube 4 is sealed. In this case, the opening of the distal end cannot be considered as overlapping the movement locus K of each of the inner through-holes 41 and 42 of the inner tube 4 and the function is different from that of the outer through-hole 51.

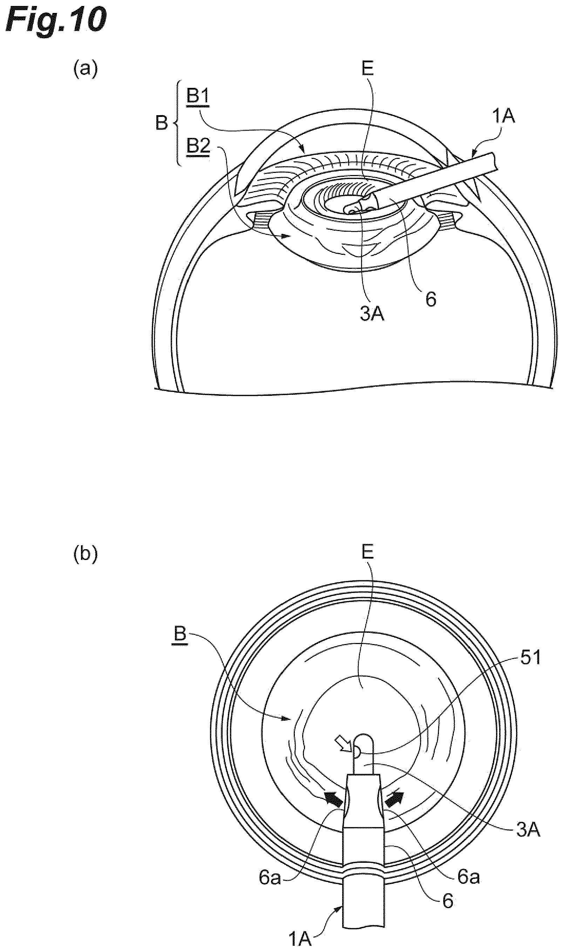

The distal end 4a of the inner tube 4 is curved and sealed and the inner through-holes 41 and 42 are provided in a side portion 4b in the vicinity of the distal end 4a of the inner tube 4. The inner through-holes 41 and 42 are provided as two types, the first inner through-hole 41 has a true circular shape, and the second inner through-hole 42 has a substantially oval shape which is elongated in the direction of the axis Sf. The diameter of the first inner through-hole 41 is substantially the same as the short diameter of the second inner through-hole 42 and hence the area of the second inner through-hole 42 is larger than that of the first inner through-hole 41. The inner through-holes 41 and 42 penetrate the inner tube 4 to enable the inside and the outside to communicate. By the driving of a suction device (not shown) connected to the I/A handpiece 1A, for example, the suction liquid La containing cortex inside the atrioventricular chamber B (see FIG. 10) passes.

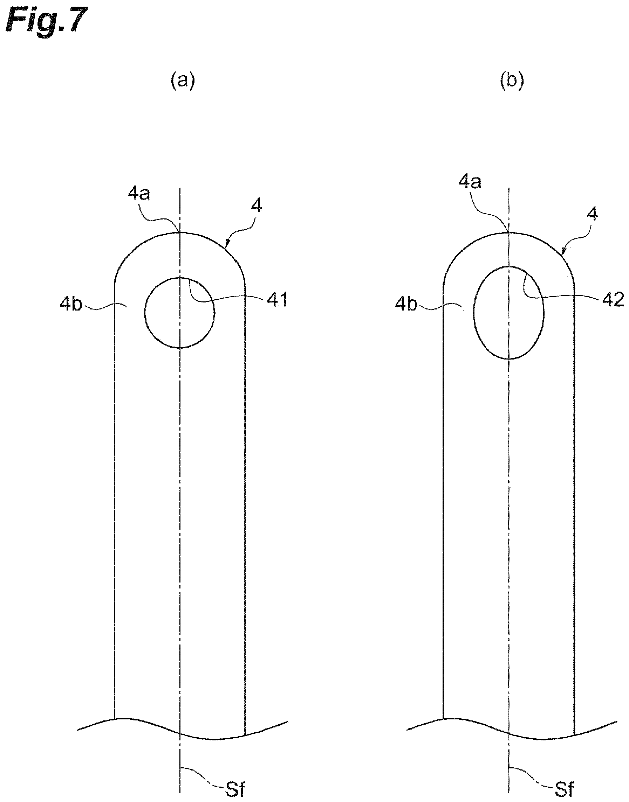

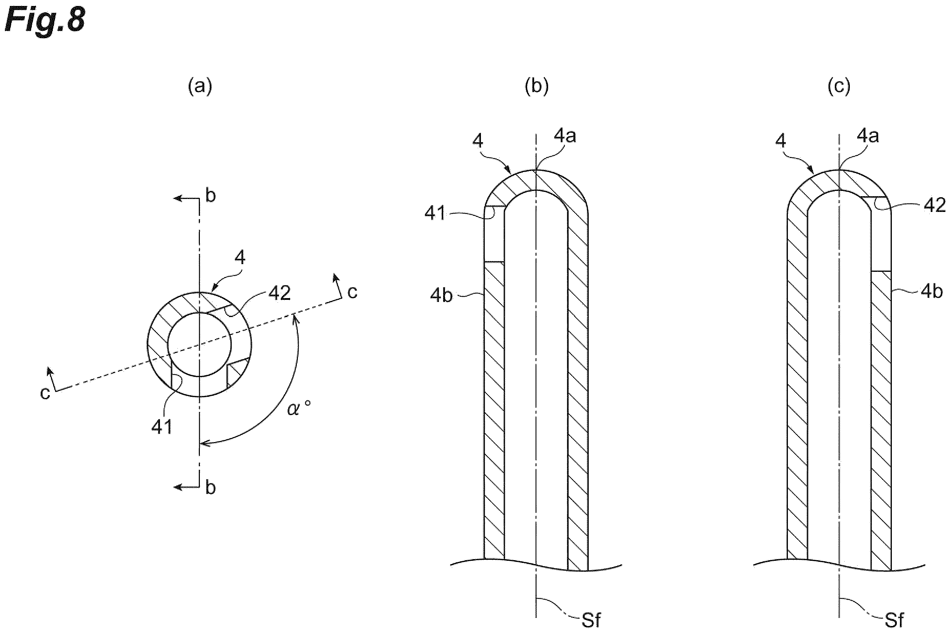

The positions of the center of the first inner through-hole 41 and the center of the second inner through-hole 42 in the direction of the axis Sf (the distance from the distal end 4a) are substantially the same (see FIGS. 7(a) and 7(b) and FIGS. 8(b) and 8(c)). Further, the center of the first inner through-hole 41 and the center of the second inner through-hole 42 deviate from each other by a phase angle α around the axis Sf (see FIG 8(a)). The phase angle α can be arbitrarily set in response to the inner diameter or shape of each of the inner through-holes 41 and 42 and further the operability for fine adjustment, but can be set, for example, in the range of 60° to 180° and the range of 90° to 110°.

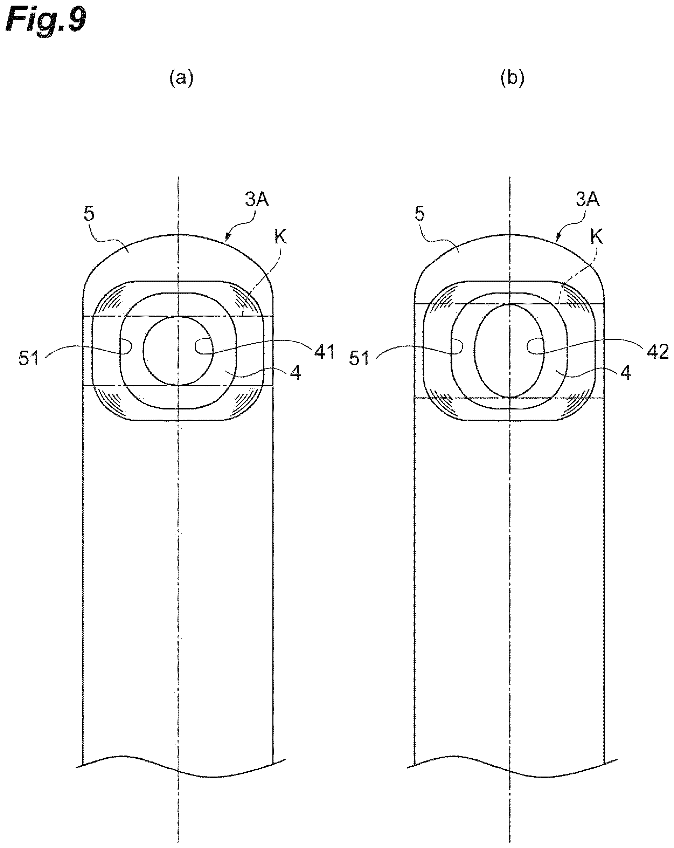

As shown in FIG 9, the outer through-hole 51 is disposed to overlap the movement locus K of each of the first inner through-hole 41 and the second inner through-hole 42 provided in the rotating inner tube 4. The overlapping with the movement locus K means a case in which at least a part of the outer through-hole 51 overlaps at least a part of the movement region when the locus (the movement region) of the first inner through-hole 41 or the second inner through-hole 42 moving along with the rotation of the inner tube 4 is assumed.

The outer through-hole 51 has a substantially square shape in which four corners are curved and the area is larger than those of the first inner through-hole 41 and the second inner through-hole 42. Further, the first inner through-hole 41 is housed in the outer through-hole 51 while the outer through-hole 51 and the first inner through-hole 41 completely overlap each other. Further, the second inner through-hole 42 is housed in the outer through-hole 51 while the outer through-hole 51 and the second inner through-hole 42 completely overlap each other. Furthermore, a case in which the first inner through-hole 41 or the second inner through-hole 42 is housed in the outer through-hole 51 means an embodiment in which the first inner through-hole 41 or the second inner through-hole 42 is completely exposed through the outer through-hole 51 and also includes, for example, an embodiment in which the shapes and the inner diameters are the same and the outer edges overlap each other in a perfect overlapping state. The shape of the outer through-hole 51 or the inner through-holes 41 and 42 is merely an example and may be other various shapes as long as the outer through-hole 51 is disposed to overlap the movement locus K of each of the inner through-holes 41 and 42. For example, the outer through-hole 51 can be formed in a circular or oval shape and conversely the inner through-holes 41 and 42 may be formed in a substantially square shape.

Next, the function and effect of the I/A handpiece 1A according to the embodiment will be described. First, a simple flow of cataract surgery (lens reconstruction technique) will be roughly described. The tissue of the eye consists of three membranes and constituents of the eyeball. The eye size is about 24 mm. The outermost side of the three membranes consists of a "cornea" and a "sclera". The middle membrane is an uvule consists of a "choroid", a "ciliary body", and an "iris". Further, the innermost membrane is a "retina". The constituents of the eyeball are various tissues inside the eyeball and fulfil functions such as: a that of a lens; maintaining shape of an eyeball; and adjusting the amount of light taken in. Furthermore, the atrioventricular chamber B includes an anterior chamber B1 and a posterior chamber B2 and a crystalline lens E is inside the posterior chamber B2.

In the lens reconstruction technique, anesthesia is first given. Anesthesia is a topical anesthesia such as eye drops or sub-Tenon's anesthesia that brings a needle point behind an eye. Next, a conjunctiva is opened and bleeding is stopped. Next, a straight knife is applied to the sclera perpendicularly, a first face is cut, and then a tunnel is made in the sclera with a crescent knife with a rounded tip. This is a second face cut. In this state, the inside of the eye is not cut. Next, a side port for cutting an anterior capsule and inserting and extracting an auxiliary tool is cut by using a straight knife.

Next, a viscoelastic substance is injected to form and hold the space of the anterior chamber B1 and then anterior capsulotomy is performed with a needle called a cystotome. Next, a third face is cut using a slit knife, which is a knife like a spear. At this time, the inside and the outside are connected. A membrane called the capsule of the crystalline lens E and the cortex are separated from each other by water so that an operation is easily performed. In this state, if the viscoelastic substance has escaped from the anterior chamber Bl, a second viscoelastic substance is injected for the purpose of protecting the corneal endothelium.

Subsequently, a lens nucleus is crushed by using an ultrasonic handpiece. When the lens nucleus is crushed completely, the ultrasonic handpiece is extracted from the atrioventricular chamber B and the distal end of the nozzle portion 3A of the I/A handpiece 1A according to the embodiment is inserted into the atrioventricular chamber B instead (see FIGS. 10(a) and 10(b)). Here, the perfusion liquid Lb is delivered to the atrioventricular chamber B and the suction liquid La is sucked along with the cortex instead.

Here, since the remaining amount of cortex is large and the viscosity is large in an initial state in which a large amount of cortex is included in the atrioventricular chamber B, the resistance during suction tends to increase. Therefore, a practitioner selects, for example, the second inner through-hole 42 having an area larger than that of the first inner through-hole 41 and promptly completes initial suction while increasing the suction amount by adjusting the handle portion 12 so that the second inner through-hole 42 overlaps the outer through-hole 51.

As the suction progresses to some extent, the remaining amount of cortex decreases and the state reaches a final suction stage. In this state, for example, a finishing procedure in which a suction hole is narrowed and suction is performed at an optimum flow rate is suitable. Therefore, the practitioner performs a procedure while decreasing the suction amount by adjusting the handle portion 12 so that the first inner through-hole 41 overlaps the outer through-hole 51.

Furthermore, the adjustment of the first inner through-hole 41 and the second inner through-hole 42 is merely an example and can be appropriately selected in consideration of the state of the liquid in the atrioventricular chamber B, the convenience and preference for the practitioner, and other operability. Further, since the suction amount changes depending on the overlapping state between the first inner through-hole 41 or the second inner through-hole 42 and the outer through-hole 51, it is possible to perform an operation of suppressing the suction amount, for example, in a state in which the first inner through-hole 41 and the outer through-hole 51 do not completely overlap each other and partially overlap each other. That is, the plurality of inner through-holes 41 and 42 may not be essentially provided in the inner tube 4, but one inner through-hole may be provided.

Here, for example, a case of using an I/A handpiece (hereinafter, referred to as a "comparative example") which cannot adjust a suction amount will be examined for the comparison with the I/A handpiece 1A according to the embodiment. Furthermore, there has been no concept conventionally of adjusting, for example, the suction amount at the initial suction and the finishing suction, nor was there any presumption of problems with using comparative examples, in particular.

In the comparative example, for example, when priority is given to the initial suction, an I/A handpiece of which a nozzle distal end has a large suction hole needs to be used. However, in this kind of I/A handpiece, there is a possibility that the trouble of the finishing treatment may cause inconvenience. Meanwhile, when priority is given to the finishing suction, the time involving with the initial suction largely increases and hence a burden of a patient increases. Particularly, in the cataract surgery (lens reconstruction technique), a burden on the patient is very large even when the difference is only about several tens of seconds.

Meanwhile, in order to reduce such a burden in time, a method can be supposed in which a plurality of comparative examples having different suction hole diameters are prepared and are separately used for the initial suction or the finishing suction. However, when the suction hole diameters are separately used, there is a need to frequently insert and extract the nozzle into and from the atrioventricular chamber B and hence a burden on the patient increases with different aspects.

In contrast tothis comparative example, according to the I/A handpiece 1A of the embodiment, it is possible to simply adjust the suction amount just by operating the handle portion 12. That is, it is possible to adjust a suction amount depending on a situation without requiring an excessive burden of the patient. As a result, it is advantageous in shortening the treatment time and it is also advantageous from the aspect of appropriate suction treatment of the cortex. Particularly, in the embodiment, since the first inner through-hole 41 and the second inner through-hole 42 having different sizes are provided, it is possible to easily adjust the suction amount in a gradual manner. Accordingly, operability is high as compared with a case in which a single inner through-hole is provided.

When the suction of cortex is completed, the process proceeds to a process of implanting an intraocular lens. In this process, a third viscoelastic substance is injected in order to form a sufficient space in the capsule enough for implanting the intraocular lens. Next, the intraocular lens is fixed into the capsule and the viscoelastic substance inside the eye is sucked and removed. Also in this suction and removal, it is possible to adjust a suction amount depending on a situation by using the I/A handpiece 1A according to the embodiment.

As described above, according to the I/A handpiece 1A of the embodiment, the overlapping region between the outer through-hole 51 and the inner through-hole can be changed by relatively moving the outer tube 5 and the inner tube 4. The suction amount increases when the overlapping region is increased. In contrast, the suction amount decreases when the overlapping region is decreased. That is, it is possible to adjust a suction amount depending on a situation without requiring an excessive burden of a patient by the adjustment of the overlapping region.

Further, in the embodiment, at least a part of the outer through-hole 51 is disposed to overlap the movement locus K of each of the inner through-holes 41 and 42. That is, since it is possible to reliably form an overlapping region with respect to at least a part of the outer through-hole 51 and to easily change the overlapping region by moving the inner through-holes 41 and 42, the adjustment is easy. Particularly, in the embodiment, since the inner through-holes 41 and 42 are moving objects, it is possible to adjust the suction amount just by moving the inner tube 4 without moving the outer tube 5 and to further reduce a burden of a patient.

Further, in the embodiment, the outer through-hole 51 is provided in the side portion 5b of the outer tube 5 and the inner through-hole is provided at the side portion 4b of the inner tube 4. That is, it is possible to effectively adjust the suction and the suction amount of the suction liquid La from the side portions 4b and 5b instead of the distal end sides of the outer tube 5 and the inner tube 4.

Further, in the embodiment, the outer tube 5 and the inner tube 4 have a common axis Sf and include a grip portion (a holding portion) 2 which holds the outer tube 5 and the inner tube 4 to be relatively movable in the rotation direction with respect to the axis Sf. That is, in the embodiment, it is possible to adjust the suction amount by the relative movement in the rotation direction instead of the direction of the axis Sf. As a result, since a design burden in consideration of at least the movement amount in the direction of the axis Sf is not necessary, it is advantageous for a compact size in the direction of the axis Sf.

Further, in the embodiment, the first inner through-hole 41 and the second inner through-hole 42 are housed in the outer through-hole 51 in a complete overlapping state. The maximum suction amount of the I/A handpiece 1A is dependent on the diameter of the inner tube 4. However, since the first inner through-hole 41 or the second inner through-hole 42 is housed in the outer through-hole 51 while overlapping the outer through-hole 51, it is possible to appropriately obtain a desired maximum suction amount in response to the diameter of the inner tube 4 without any disturbance of the outer tube 5.

Further, the movable body 7 according to the embodiment is provided with the handle portion 12 which protrudes in a direction orthogonal to the axis Sf and penetrates the casing portion 8 to be exposed from the casing portion 8. As a result, since it is possible to simply adjust the suction amount by rotating the handle portion 12, it is possible to improve operability.

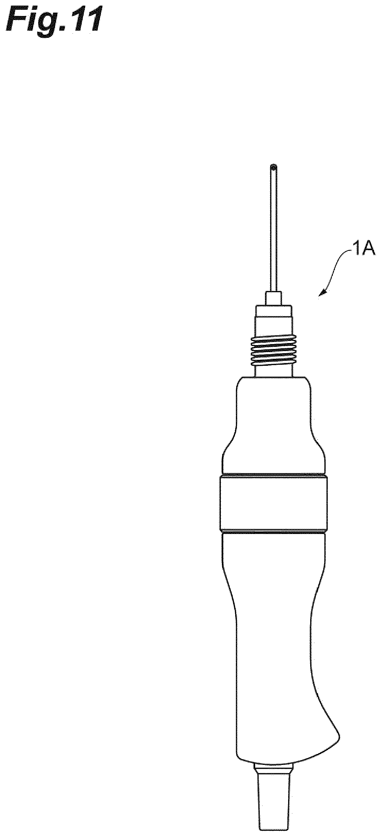

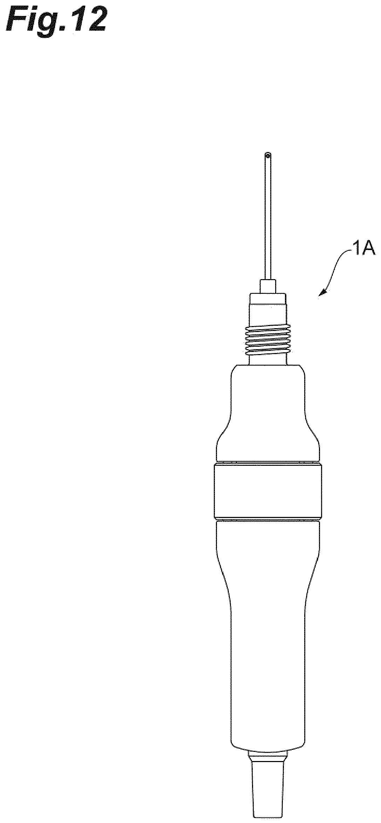

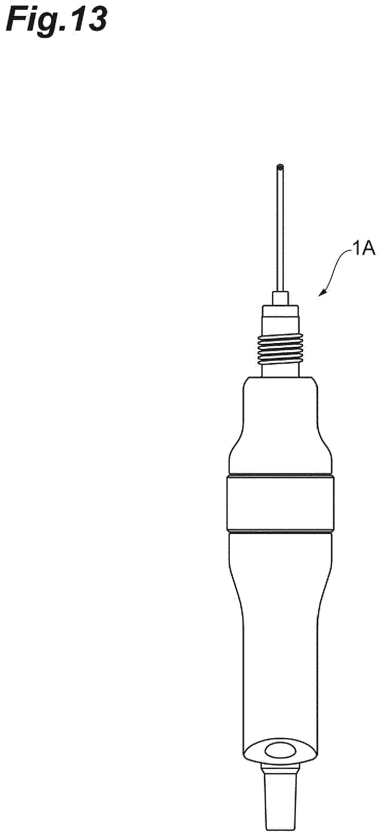

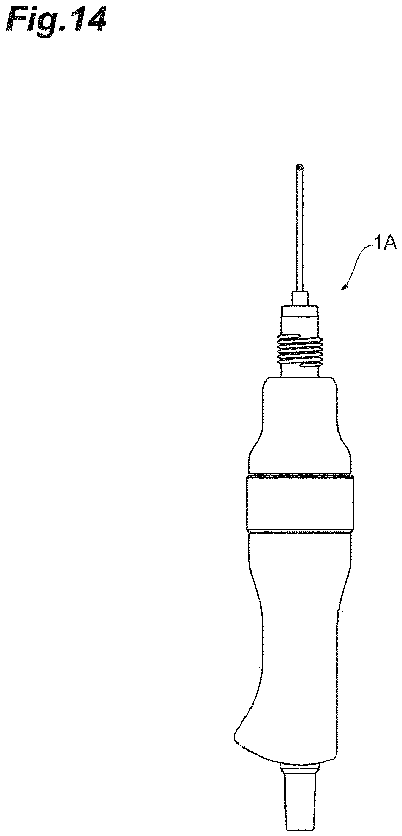

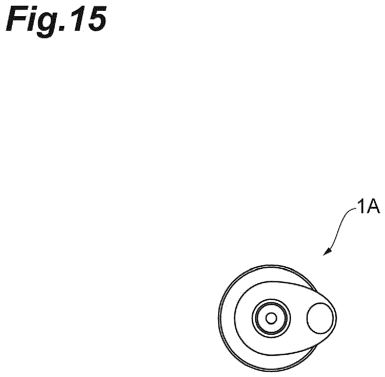

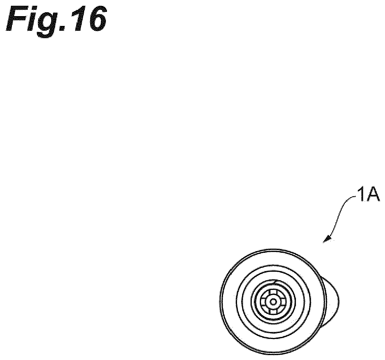

Furthermore, FIGS. 11 to 16 are diagrams showing the I/A handpiece 1A according to the above-described embodiment when viewed from six sides, where FIG 11 is a front view, FIG 12 is a left view, FIG 13 is a right view, FIG 14 is a rear view, FIG. 15 is a bottom view, and FIG. 16 is a plan view (a top view).

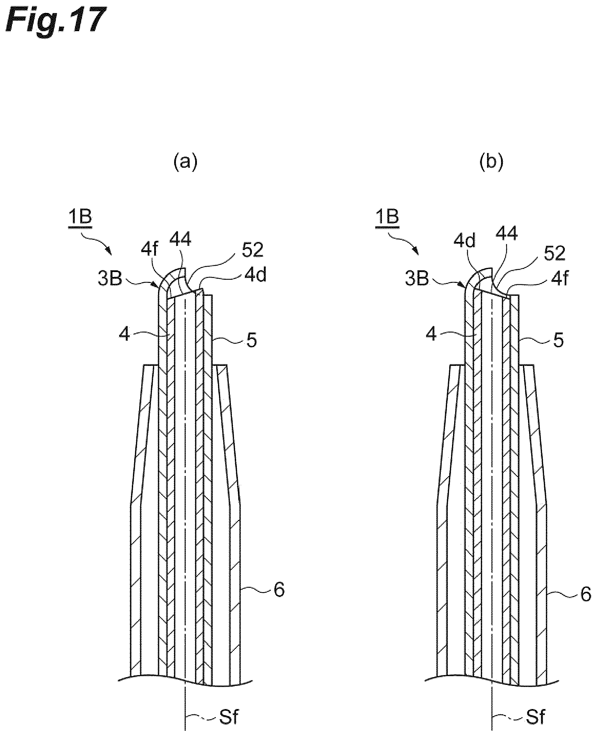

Next, an I/A handpiece 1B according to a second embodiment will be described with reference to FIG. 17. The I/A handpiece 1B according to the embodiment basically has the same component or structure as that of the I/A handpiece 1A according to the first embodiment. Thus, in the description below, a difference from the I/A handpiece 1A according to the first embodiment will be mainly described. Then, the same reference numerals will be given to the same component or structure and a detailed description and the like thereof will be omitted.

The I/A handpiece 1B includes a grip portion (a holding portion) 2 which is gripped by a practitioner, a nozzle portion 3B through which a liquid for perfusion passes, and a sleeve 6 which guides to the distal end side of the nozzle portion 3B a liquid (a perfusion liquid) Lb fed into the I/A handpiece 1B and discharged through the grip portion 2.

The nozzle portion 3A is a double tube and includes an inner tube 4 which is provided with an inner through-hole 44 and an outer tube 5 which accommodates the inner tube 4 and is provided with an outer through-hole 52. In the embodiment, the inner tube 4 fixed to a movable body 7 is rotatable with respect to the outer tube 5 fixed to a casing portion 8.

A distal end of the inner tube 4 is of a shape such that it was cut at an inclination surface with respect to the axis Sf of the inner tube 4 and the distal end is provided with the inner through-hole 44. The outer tube 5 is of a shape such that half of the distal end is cut away and the outer through-hole 52 is formed at the distal end similarly to the inner tube 4. The distal end of the inner tube 4 provided with the inner through-hole 44 includes an acute angle portion 4d which protrudes along the direction of the axis Sf and an obtuse angle portion 4f which is located at the opposite side. When the inner through-hole 44 rotates around the axis Sf so that the acute angle portion 4d overlaps the outer through-hole 52, the acute angle portion 4d protrudes to block a part of the outer through-hole 52 (see FIG. 17(a)). In contrast, when the obtuse angle portion 4f overlaps the outer through-hole 52, the obtuse angle portion 4f is retracted to avoid the outer through-hole 52 (see FIG. 17(b)). As a result, when the acute angle portion 4d overlaps the outer through-hole 52, the overlapping region between the inner through-hole 44 and the outer through-hole 52 decreases. Then, when the obtuse angle portion 4f overlaps the outer through-hole 52, the overlapping region between the inner through-hole 44 and the outer through-hole 52 increases.

That is, according to the embodiment, it is possible to change the overlapping region between the outer through-hole 51 and the inner through-hole 44 by relatively rotating the inner tube 4 with respect to the outer tube 5. It is possible to adjust a suction amount depending on a situation without requiring an excessive burden of a patient by adjusting the overlapping region. Further, according to the embodiment, the same function and effect based on the same component or structure as that of the I/A handpiece 1A according to the first embodiment can be obtained.

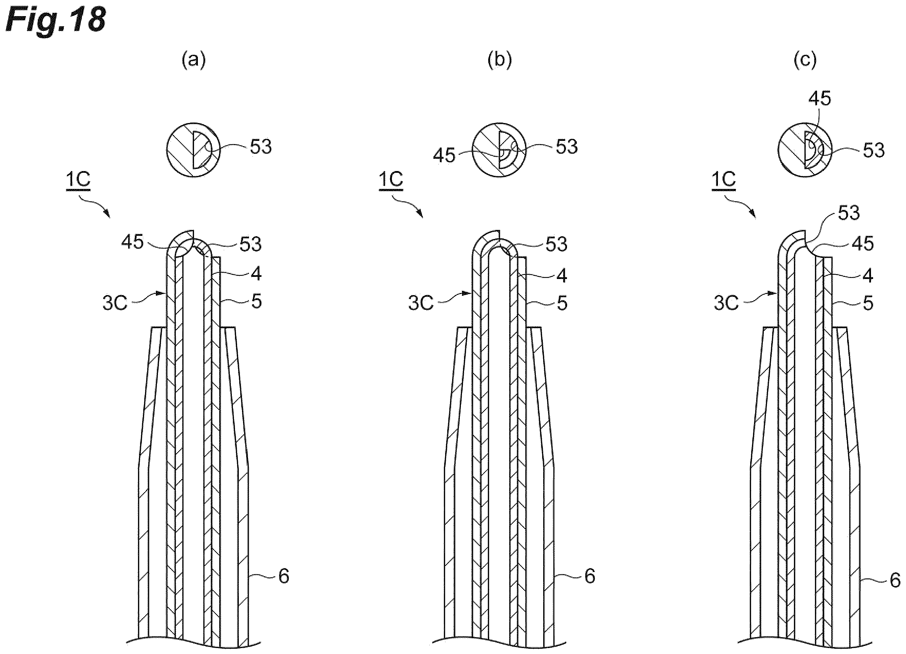

Next, an I/A handpiece 1C according to a third embodiment will be described with reference to FIG. 18. The I/A handpiece 1C according to the embodiment basically has the same component or structure as that of the I/A handpieces 1A or 1B according to the first or second embodiment. Thus, in the description below, a difference from the I/A handpiece 1A or 1B according to the first or second embodiment will be mainly described. Then, the same reference numerals will be given to the same component or structure and a detailed description and the like thereof will be omitted.

The I/A handpiece 1C includes a grip portion (a holding portion) 2 which is gripped by a practitioner, a nozzle portion 3C through which a liquid for perfusion passes, and a sleeve 6 which guides to the distal end side of the nozzle portion 3C a liquid (a perfusion liquid) Lb fed into the I/A handpiece 1C and discharged through the grip portion 2.

The nozzle portion 3C is a double tube and includes an inner tube 4 which is provided with an inner through-hole 45 and an outer tube 5 which accommodates the inner tube 4 and is provided with an outer through-hole 53. In the embodiment, the inner tube 4 fixed to a movable body 7 is rotatable with respect to the outer tube 5 fixed to a casing portion 8.

The inner tube 4 is of a shape such that half of the distal end is cut and the inner through-hole 45 is formed in the distal end into a semi-circular shape. The outer tube 5 is of a shape such that half of the distal end is cut away and the outer through-hole 53 is formed at the distal end similarly to the inner tube 4. In the embodiment, at least a part of the outer through-hole 53 is disposed to overlap the movement locus of the inner through-hole 45. Specifically, in a state in which the inner through-hole 45 is hidden behind the distal end of the outer tube 5, an overlapping region is not formed between the inner through-hole 45 and the outer through-hole 53 (see FIG 18(a)). Next, when the inner tube 4 rotates by 90° in the clockwise rotation direction in plan view (see FIG. 18(b)), the half of the inner through-hole 45 overlaps the outer through-hole 53 and hence a half overlapping region is formed. Further, when the inner tube 4 rotates by 90° in the clockwise rotation direction (see FIG. 18(c)), the inner through-hole 45 and the outer through-hole 53 completely overlap each other and hence the inner through-hole 45 is housed in the outer through-hole 53. In a state in which the inner through-hole 45 is housed in the outer through-hole 53, the overlapping region increases as compared with the half overlapping region.

That is, according to the embodiment, it is possible to change the overlapping region between the outer through-hole 53 and the inner through-hole 45 by relatively rotating the inner tube 4 with respect to the outer tube 5. It is possible to adjust a suction amount depending on a situation without requiring an excessive burden of a patient by adjusting the overlapping region. Further, according to the embodiment, the same function and effect based on the same component or structure as that of the I/A handpiece 1A according to the first embodiment can be obtained.

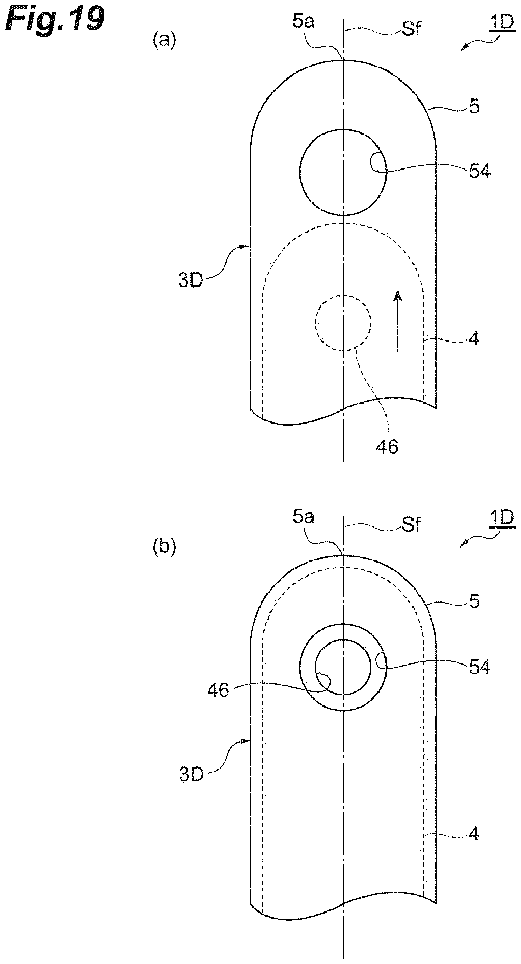

Next, an I/A handpiece 1D according to a fourth embodiment will be described with reference to FIG 19. The I/A handpiece 1D according to the embodiment basically has the same component or structure as that of the I/A handpieces 1A, 1B, and 1C according to the first to third embodiments. Thus, in the description below, a difference from the I/A handpieces 1A, 1B, and 1C according to the first to third embodiments will be mainly described. Then, the same reference numerals will be given to the same component or structure and a detailed description and the like thereof will be omitted.

The I/A handpiece 1D includes a grip portion (a holding portion) 2 which is gripped by a practitioner, a nozzle portion 3D through which a suction liquid La passes, and a sleeve 6 which guides to the distal end side of the nozzle portion 3D a liquid (a perfusion liquid) Lb fed into the I/A handpiece 1D and discharged through the grip portion 2.

The nozzle portion 3D is a double tube and includes an inner tube 4 which is provided with an inner through-hole 46 and an outer tube 5 which accommodates the inner tube 4 and is provided with an outer through-hole 54. In the embodiment, the inner tube 4 fixed to a movable body 7 is relatively slidable along the direction of the axis Sf with respect to the outer tube 5 fixed to a casing portion 8.

A side portion 4b of the inner tube 4 is provided with an inner through-hole 46 and a side portion 5b of the outer tube 5 is provided with an outer through-hole 54. In the embodiment, at least a part of the outer through-hole 54 is disposed to overlap the movement locus K of the inner through-hole 46 moving along the direction of the axis Sf. Specifically, in a state in which the inner through-hole 46 is separated from the distal end of the outer tube 5, the inner through-hole 46 is hidden behind the outer tube 5 and an overlapping region is not formed between the inner through-hole 46 and the outer through-hole 51 (see FIG 19(a)). Next, when the inner tube 4 slides so that the inner through-hole 46 moves closer to the distal end of the outer tube 5 and reaches a predetermined position, the inner through-hole 46 and the outer through-hole 54 completely overlap each other and hence the inner through-hole 46 is housed in the outer through-hole 51. In a state in which the inner through-hole 46 is housed in the outer through-hole 51, the overlapping region increases as compared with an intermediate state until the inner through-hole reaches a predetermined position.

That is, according to the embodiment, it is possible to change the overlapping region between the outer through-hole 51 and the inner through-hole 46 by relatively sliding the inner tube 4 with respect to the outer tube 5. As a result, it is possible to adjust a suction amount depending on a situation without requiring an excessive burden of a patient by adjusting an overlapping region. Further, according to the embodiment, the same function and effect based on the same component or structure as that of the I/A handpiece 1A according to the first embodiment can be obtained.

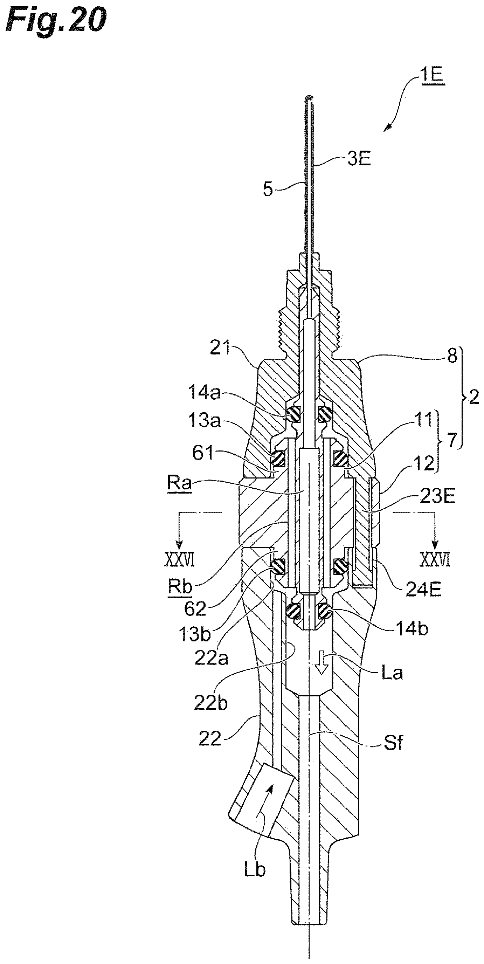

Next, an I/A handpiece 1E according to a fifth embodiment will be described with reference to FIGS. 20 to 27. The I/A handpiece 1E basically has the same component or structure as that of the I/A handpiece 1A according to the first embodiment. Thus, in the description below, a difference from the I/A handpiece 1A according to the first embodiment will be mainly described. Then, the same reference numerals will be given to the same component or structure and a detailed description and the like thereof will be omitted. Furthermore, in the description below, the axial direction means a direction along the axis Sf.

As shown in FIG. 20, the I/A handpiece 1E includes a grip portion (a holding portion) 2 which is gripped by a practitioner, a nozzle portion 3E through which a liquid for perfusion passes, and a sleeve 6 (not shown in FIG. 20) which guides to the distal end side of the nozzle portion 3E a liquid (a perfusion liquid) Lb fed into the I/A handpiece 1E and discharged through the grip portion 2.

The grip portion 2 includes a movable body 7 to which the inner tube 4 of the nozzle portion 3E is fixed and a casing portion 8 to which the outer tube 5 of the nozzle portion 3E is fixed. The casing portion 8 is separable from the movable body 7, accommodates the movable body 7, and rotatably supports the movable body 7. The movable body 7 is provided with an inner passage Ra which communicates with the inner tube 4 and an outer passage Rb through which a perfusion liquid Lb passes.

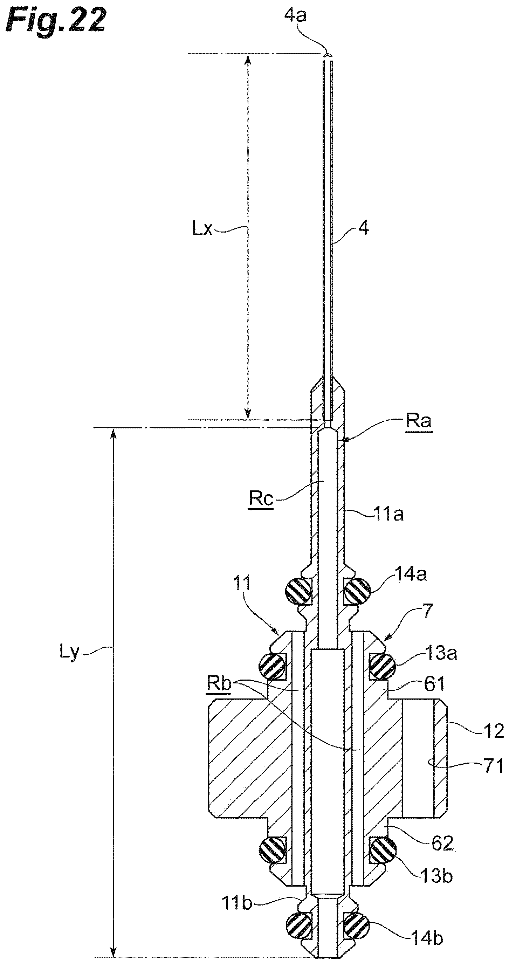

The inner passage Ra is provided with an enlarged passage portion Rc of which an inner diameter is larger than the inner diameter of the inner tube 4 (see FIG 22). A length Ly of the enlarged passage portion Rc in the axial direction is longer than a length Lx of the inner tube 4 in the axial direction.

The movable body 7 is provided with a tubular body portion 11 and a handle portion 12 which protrudes from the body portion 11 in the radial direction. The body portion 11 is provided with a first protrusion portion 61 which protrudes from the handle portion 12 to the distal end side and a second protrusion portion 62 which protrudes to the base end side. The first protrusion portion 61 and the second protrusion portion 62 protrude in the axial direction. The first protrusion portion 61 is an example of a "protrusion shaft body".

An O-ring (an annular seal member) 13a is attached to the first protrusion portion 61 and an O-ring 13b is attached to the second protrusion portion 62. Further, a distal end tube 11a of a small diameter protrudes to the distal end side of the first protrusion portion 61 and a base end tube 11b having a small diameter protrudes to the second protrusion portion 62. The inner tube 4 is fixed to the distal end tube 11a.

The casing portion 8 is provided with a discharge side casing (a first casing) 21 which is disposed at the distal end side for discharging the perfusion liquid Lb and an introduction side casing (a second casing) 22 which is disposed at a base end side for receiving the perfusion liquid Lb. The introduction side casing 22 is disposed opposite the discharge side casing 21 with the handle portion 12 interposed therebetween.

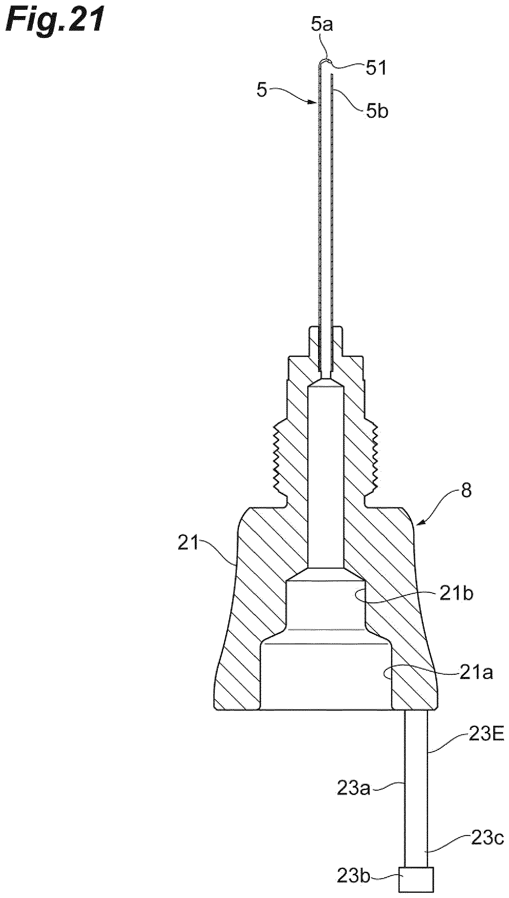

The discharge side casing 21 (see FIG. 21) is provided with a discharge side concave portion (a concave portion) 21a which accommodates a first protrusion portion 61 of the body portion 11 and an inner tube insertion hole 21b which communicates with the center of the discharge side concave portion 21a. The O-ring 13a is in contact with the discharge side concave portion (the concave portion) 21a and the O-ring 14a is in contact with the inner tube insertion hole 21b. In the embodiment, the discharge side concave portion 21a is an example of a "concave portion which accommodates a protrusion shaft body and is in contact with a seal member". Further, a discharge passage 21c (see FIG 3) is provided in the discharge side casing 21 so as to avoid the inner tube insertion hole 21b, and the discharge passage connects a discharge port 2c (see FIGS. 1 and 5) for the perfusion liquid Lb and the discharge side concave portion 21a to communicate. Furthermore, the discharge passage 21c is omitted in FIGS. 20 and 21 for convenience of description.

The introduction side casing 22 is provided with an introduction side concave portion 22a which accommodates a second protrusion portion 62 of the body portion 11 and a suction path 22b which communicates with the center of the introduction side concave portion 22a. The O-ring 13b is in contact with the introduction side concave portion 22a and the O-ring 14b is in contact with the suction path 22b.

The discharge side casing 21 and the introduction side casing 22 are disposed with a gap interposed therebetween through a connection portion 23E. The discharge side casing 21 and the introduction side casing 22 are integrally coupled to each other by the fitting of the connection portion 23E and a connection hole 24E so that the casing portion 8 is formed. The connection portion 23E is a single body and is an example of a "guide rod".

The connection portion 23E is provided with a rod main body 23a which is fixed to the discharge side casing 21 and a head portion 23b which is provided in an end of the rod main body 23a. The end of the rod main body 23a refers to an end opposite a root fixed to the discharge side casing 21 in the rod main body 23a. The head portion 23b is detachably fitted to the connection hole 24E of the introduction side casing 22.

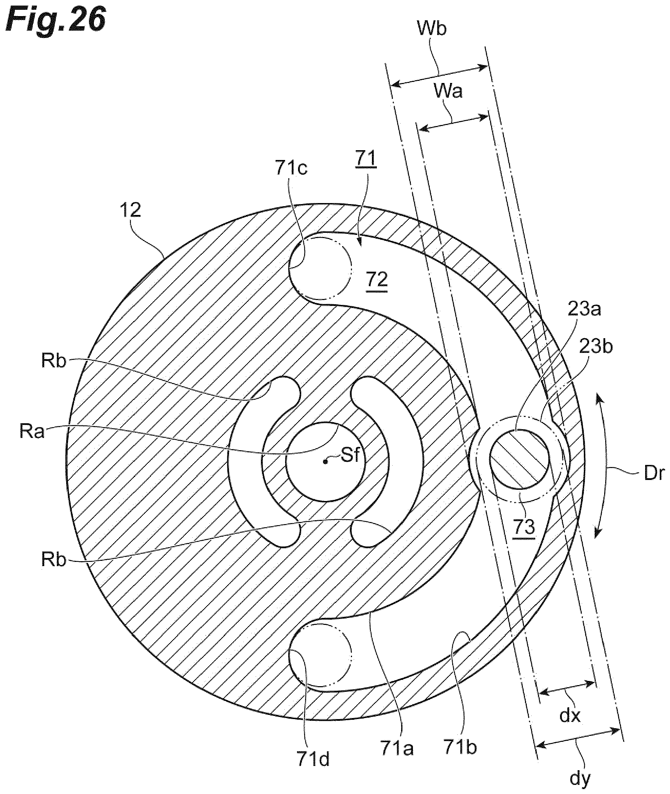

The rod main body 23a has a linear shape extending in the axial direction and is, for example, a columnar member. The head portion 23b is, for example, a columnar member and an outer diameter dy of the head portion 23b is larger than a maximum outer diameter dx of the rod main body 23a (see FIG. 26). Furthermore, in the embodiment, the outer diameter of the rod main body 23a is uniform, but may be different.

The handle portion 12 (see FIGS. 22 and 26) is provided with an escaping hole (a guide hole) 71 which penetrates in the axial direction and is elongated along a rotation direction Dr around the axis Sf when viewed from the axial direction. The rod main body 23a of the connection portion 23E is inserted through the escaping hole 71. The escaping hole 71 is a hole for preventing interference between the handle portion 12 and the rod main body 23a of the connection portion 23E. The escaping hole 71 is provided to correspond to the number of the connection portions 23E.

The escaping hole 71 is formed by a first guide surface 71a, a second guide surface 71b, and a pair of stopper surfaces 71c and 71d. The first guide surface 71a is a surface which extends along the rotation direction Dr around the axis Sf. The second guide surface 71b is a surface which is away from the axis Sf in relation to the first guide surface 71a and extends while facing the first guide surface 71a. The pair of stopper surfaces 71c and 71d are surfaces which are provided at both ends of the first guide surface 71a and the second guide surface 71b and come into contact with the rod main body 23a of the connection portion 23E to regulate the relative movement of the connection portion 23E.

The escaping hole 71 includes a first region 72 and a second region 73. The first region 72 is a region in which a distance Wa between the first guide surface 71a and the second guide surface 71b is larger than a maximum outer diameter dx of the rod main body 23a and is smaller than an outer diameter dy of the head portion 23b. The second region 73 is a region in which a distance Wb between the first guide surface 71a and the second guide surface 71b is larger than an outer diameter dy of the head portion 23b. Further, the second region 73 is narrower than the first region 72 in the rotation direction Dr around the axis Sf. In the embodiment, the second region is provided at the substantially center portion of the first region 72 in the rotation direction Dr. Further, the second region 73 extends in a linear shape along the axis Sf.

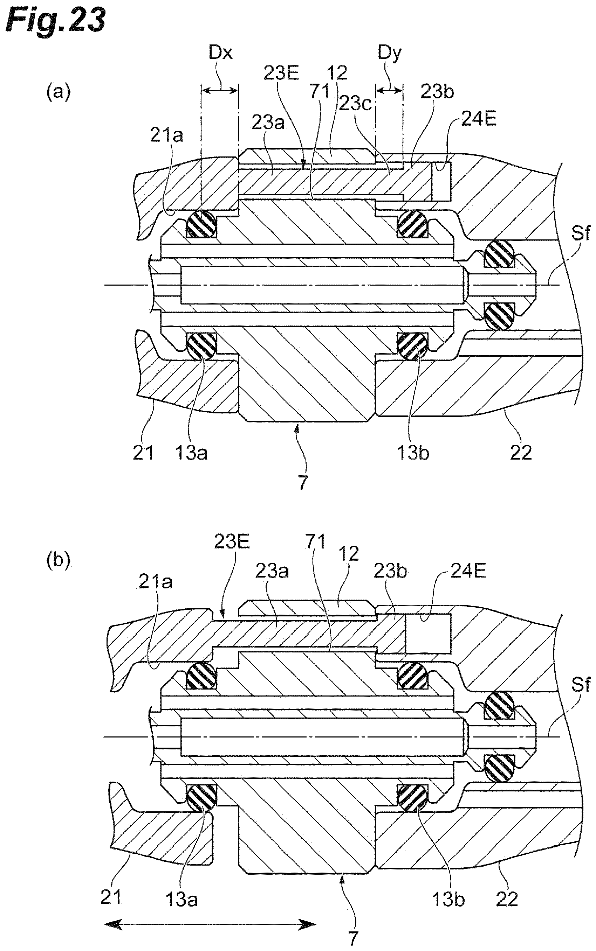

The length of the escaping hole 71 in the axial direction, that is, the thickness of the handle portion 12 (see FIG. 23) is shorter than the length of the rod main body 23a in the axial direction. Thus, a part of the rod main body 23a on a head portion 23b side protrudes, the rod main body 23a being inserted through the escaping hole 71. This part is a remaining portion 23c of the rod main body 23a. A gap is formed between the handle portion 12 and the head portion 23b by the remaining portion 23c.

Further, a distance Dx from the O-ring 13a to an end portion near the handle portion 12 of the discharge side concave portion (the concave portion) 21a is equal to or longer than a length Dy of the remaining portion 23c in the axial direction. Furthermore, the distance Dx substantially means a distance from a position corresponding to the center in the axial direction of the contact portion between the O-ring 13a and the discharge side concave portion (the concave portion) 21a to an end portion near the handle portion 12 of the discharge side concave portion 21a.

In general, when the movement of the discharge side casing deviates from the axial direction at the time of separating the discharge side casing from the movable body, there is a possibility that the inner tube may interfere with the outer tube so that the inner tube is damaged. This deviation easily occurs in moments when a load applied to the O-ring is released due to the O-ring separating from the concave portion of the first casing.

In contrast, in the embodiment, when the discharge side casing 21 is moved by the length Dy of the remaining portion 23c of the rod main body 23a with respect to the movable body 7, the head portion 23b of the connection portion 23E reaches the escaping hole 71 (see FIG. 23(b)). The O-ring 13a near the movable body 7 moves in the opposite direction in response to this movement. Here, the distance Dx from the O-ring 13a to an end portion near the handle portion 12 of the discharge side concave portion 21a is equal to or longer than the length Dy of the remaining portion 23c. That is, the O-ring 13a is not separated from the discharge side concave portion 21a at a timing at which the head portion 23b reaches the escaping hole 71.

Next, when the reaching position is within the first region 72 (see FIG. 26) even when the head portion 23b reaches the escaping hole 71, the head portion cannot pass through the escaping hole 71 and hence the movement of the movable body 7 is regulated. Thus, in order to further move the movable body 7, there is a need to adjust the position so that the head portion 23b reaches the second region 73 by rotating the movable body 7. Thus, in the embodiment, there is a possibility that the operator has a chance of adjusting the position of the head portion 23b before the O-ring 13a is separated from the discharge side concave portion 21a. Accordingly, it is easy to suppress the damage of the inner tube 4 by prompting a careful movement operation of the discharge side casing 21 or the like.

In particular, in the embodiment, the distance Dx from the O-ring 13a to an end portion near the handle portion 12 of the discharge side concave portion 21a is the same as the length Dy of the remaining portion 23c in the axial direction. Thus, in the embodiment, there is a possibility that the operator has a chance of adjusting the position of the head portion 23b before the O-ring 13a is separated from the discharge side concave portion 21a. Accordingly, it is easy to suppress the damage of the inner tube 4 by more appropriately prompting a careful movement operation of the discharge side casing 21 or the like.

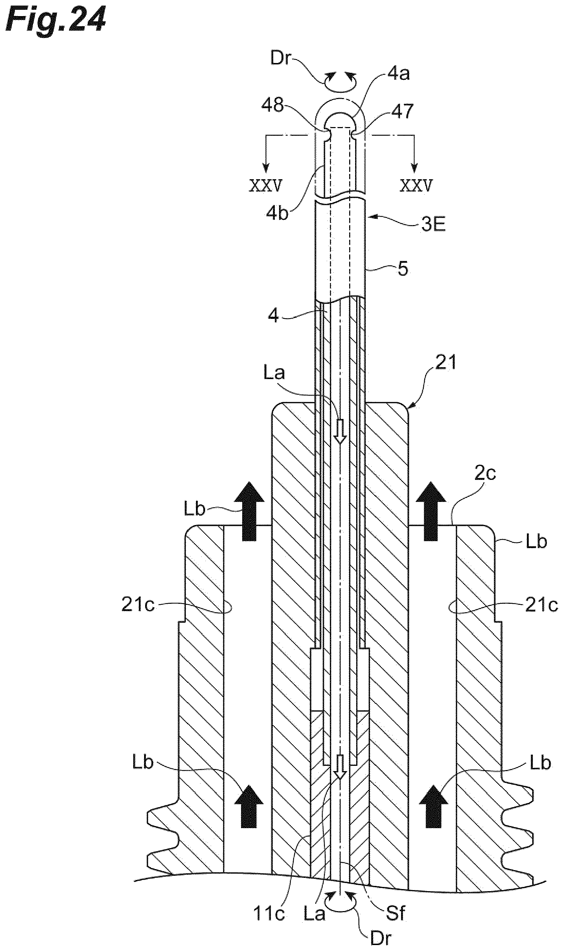

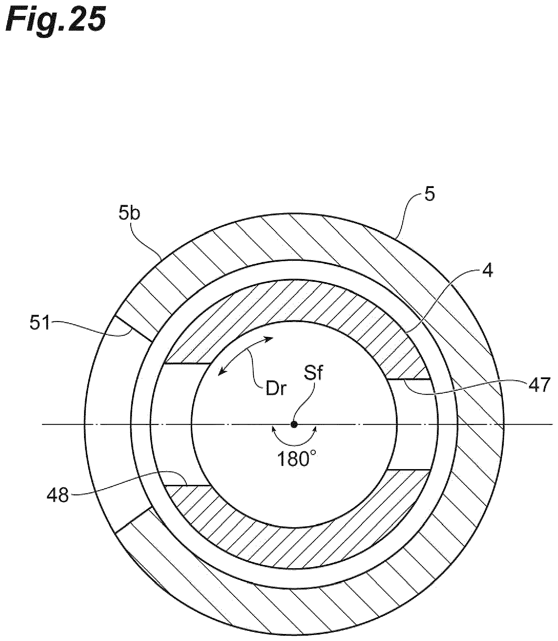

Next, the nozzle portion 3E will be described. As shown in FIGS. 24 and 25, the nozzle portion 3E is a double tube and includes an inner tube 4 and an outer tube 5 which accommodates the inner tube 4 and is provided with an outer through-hole 51. The inner tube 4 is provided with a first inner through-hole 47 and a second inner through-hole 48. A passage area of the second inner through-hole 48 is larger than that of the first inner through-hole 47. In the embodiment, the inner tube 4 fixed to the movable body 7 is relatively rotatable with respect to the outer tube 5 fixed to the casing portion 8.

The distal end 5a of the outer tube 5 is curved and sealed and the outer through-hole 51 is provided in the side portion 5b in the vicinity of the distal end 5a of the outer tube 5. The outer through-hole 51 penetrates the outer tube 5 to enable the inside and the outside to communicate.

The distal end 4a of the inner tube 4 is curved and sealed and the first inner through-hole 47 and the second inner through-hole 48 are provided in the side portion 4b in the vicinity of the distal end 4a of the inner tube 4. The first inner through-hole 47 and the second inner through-hole 48 have a true circular shape. The diameter of the first inner through-hole 47 is smaller than that of the second inner through-hole 48. Thus, the area of the second inner through-hole 48 is larger than that of the first inner through-hole 47. The first inner through-hole 47 and the second inner through-hole 48 penetrate the inner tube 4 to enable the inside and the outside to communicate.

The positions of the center of the first inner through-hole 47 and the center of the second inner through-hole 48 in the direction of the axis Sf (the distance from the distal end 4a) are substantially the same. Further, the center of the first inner through-hole 47 and the center of the second inner through-hole 48 deviate from each other by a phase angle of 180° around the axis Sf (see FIG. 25). That is, the first inner through-hole 47 and the second inner through-hole 48 are provided at a position in which a rotation angle around the axis Sf is 180°.

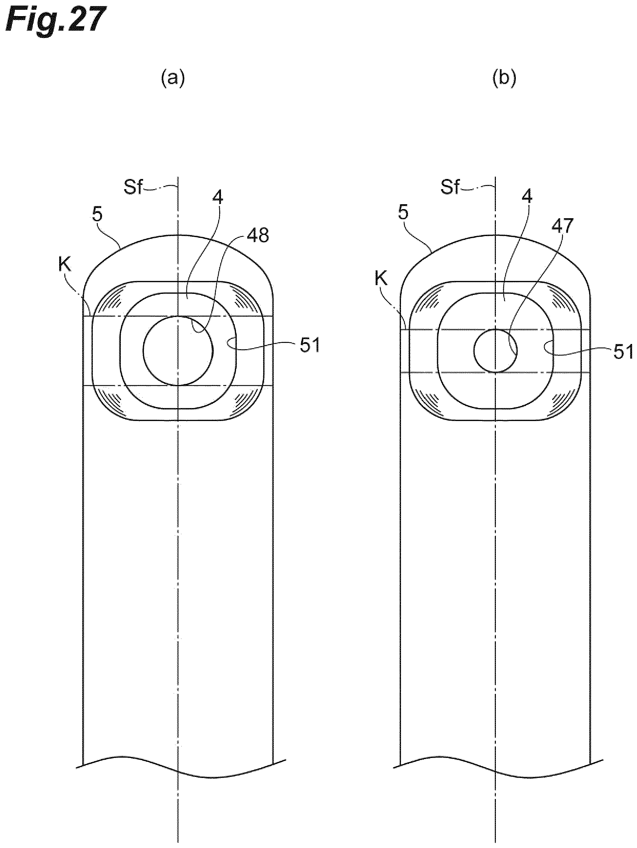

The outer through-hole 51 (see FIG. 27) is disposed to overlap the movement locus K of each of the first inner through-hole 47 and the second inner through-hole 48 provided in the rotating inner tube 4. The overlapping with the movement locus K means a case in which at least a part of the outer through-hole 51 overlaps at least a part of the movement region on the assumption that there is a locus (a movement region) of the first inner through-hole 47 or the second inner through-hole 48 moving in accordance with the rotation of the inner tube 4.

In a state in which the outer through-hole 51 and the first inner through-hole 47 completely overlap each other, the first inner through-hole 47 is housed in the outer through-hole 51. Further, in a state in which the outer through-hole 51 and the second inner through-hole 48 completely overlap each other, the second inner through-hole 48 is housed in the outer through-hole 51. The shape of each of the outer through-hole 51, the first inner through-hole 47, and the second inner through-hole 48 is merely an example and may be other various shapes as long as the outer through-hole 51 is disposed to overlap the movement locus K of each of the first inner through-hole 47 and the second inner through-hole 48. For example, the outer through-hole 51 can be formed in a circular shape or an oval shape. In contrast, the first inner through-hole 47 or the second inner through-hole 48 can be formed in a substantially square shape.

As described above, the rod main body 23a of the connection portion 23E is inserted through the escaping hole 71. When the movable body 7 is rotated with respect to the discharge side casing 21, the rod main body 23a moves inside the escaping hole 71. When the connection portion 23E (specifically, the rod main body 23a) comes into contact with the pair of stopper surfaces 71c and 71d, the movement of the movable body 7 is regulated. Here, the first inner through-hole 47 and the outer through-hole 51 overlap each other at a position in which the connection portion 23E comes into contact with one stopper surface 71c of the pair of stopper surfaces 71c and 71d. Further, the second inner through-hole 48 and the outer through-hole 51 overlap each other at a position in which the connection portion 23E comes into contact with the other stopper surface 71d.

That is, when the connection portion 23E comes into contact with one stopper surface 71c at the time of rotating the movable body 7, the first inner through-hole 47 and the outer through-hole 51 are aligned to overlap each other. Then, when the connection portion 23E comes into contact with the other stopper surface 71d, the second inner through-hole 48 and the outer through-hole 51 are aligned to overlap each other. As a result, since it is easy to align the first inner through-hole 47 and the outer through-hole 51 and to align the second inner through-hole 48 and the outer through-hole 51, operability is improved.

Further, the first inner through-hole 47 and the second inner through-hole 48 are provided at a position in which a rotation angle around the axis Sf is 180°. That is, the first inner through-hole 47 and the second inner through-hole 48 are provided at a position facing each other with reference to the axis Sf. When the first inner through-hole 47 and the second inner through-hole 48 face each other, it is easy to increase the size and to increase the flow amount of the liquid passing through the first inner through-hole 47 and the second inner through-hole 48.

According to the embodiment, at least a part of the outer through-hole 51 can overlap any one of the inner through-holes having different sizes by the rotation of the inner tube 4 with respect to the outer tube 5. For example, in the embodiment, the first inner through-hole 47 and the second inner through-hole 48 are provided. When the outer through-hole 51 overlaps the large second inner through-hole 48, the liquid suction amount increases. Meanwhile, when the outer through-hole overlaps the small first inner through-hole 47, the liquid suction amount decreases. That is, it is possible to change the overlapping region between the first inner through-hole 47 or the second inner through-hole 48 and the outer through-hole 51 by the selection of the inner through-holes 47 and 48 overlapping the outer through-hole 51. That is, it is possible to adjust a suction amount depending on a situation without requiring an excessive burden of a patient by adjusting the overlapping region. Further, according to the embodiment, the same function and effect based on the same component or structure as that of the I/A handpiece 1A according to the first embodiment can be obtained.

Further, in the embodiment, the inner passage Ra provided in the movable body 7 is provided with an enlarged passage portion Rc of which an inner diameter is larger than that of the inner tube 4 and a length Ly of the enlarged passage portion Rc in the axial direction is longer than a length Lx of the inner tube 4. Since the inner diameter of the inner tube 4 is smaller than the inner diameter of the enlarged passage portion Rc, a resistance is large in the inner tube 4 as compared with the enlarged passage portion Rc. However, since a length Ly of the enlarged passage portion Rc in the axial direction is longer than the length Lx of the inner tube 4, it is possible to decrease the resistance in all of the inner tube 4 and the inner passage Ra and it is easy to increase the flow amount of the liquid passing through the first inner through-hole 47 or the second inner through-hole 48.

Further, in the embodiment, when the connection portion (the guide rod) 23E comes into contact with one stopper surface 71c, the first inner through-hole 47 and the outer through-hole 51 are aligned to overlap each other. Then, when the connection portion 23E comes into contact with the other stopper surface 71d, the second inner through-hole 48 and the outer through-hole 51 are aligned to overlap each other. As a result, since it is easy to align the first inner through-hole 47 and the outer through-hole 51 and to align the second inner through-hole 48 and the outer through-hole 51, operability is improved.

Further, in the embodiment, the first inner through-hole 47 and the second inner through-hole 48 are provided at a position in which a rotation angle is 180° around the axis Sf. That is, the first inner through-hole 47 and the second inner through-hole 48 are provided at a position facing each other with reference to the axis Sf. As a result, since it is easy to increase the size in a range in which the first inner through-hole 47 and the second inner through-hole 48 do not interfere with each other, it is easy to increase the flow amount of the liquid passing through the first inner through-hole 47 and the second inner through-hole 48.

Further, in the embodiment, in a case in which the discharge side casing (the first casing) 21 and the introduction side casing (the second casing) 22 are separated from each other and the connection portion (the guide rod) 23E is extracted from the escaping hole (the guide hole) 71, the head portion 23b of the connection portion 23E can pass through the second region 73, but cannot pass through the first region 72. Further, since the second region 73 extends along the axis Sf, the connection portion 23E can be extracted in a direction along the axis Sf. As a result, the discharge side casing 21 can be separated in a direction along the axis Sf with respect to the movable body 7.

Although the I/A handpiece 1E according to the fifth embodiment has been described, the structure of the I/A handpiece 1E can be also applied to the I/A handpieces 1A to 1D according to the first to fourth embodiments. For example, the enlarged passage portion Rc can be formed in the inner passage Ra formed in the movable body 7 of each of the I/A handpieces 1A to 1D and the length of the enlarged passage portion Rc in the axial direction can be set to be longer than the length of the inner tube 4. Further, the connection portion 23E and the escaping hole 71 according to the fifth embodiment can be also applied to the I/A handpieces 1A to 1D according to the first to fourth embodiments.

### Examples

Hereinafter, the invention will be described in more detail with reference to examples, but the invention is not limited to these examples.

Example 1 is a result verified by comparing the suction amounts at the time of using two kinds of inner tubes applied to the I/A handpiece. The diameter of the inner through-hole (the first inner through-hole) of the first inner tube was 0.3 mm and the diameter of the inner through-hole (the second inner through-hole) of the second inner tube was 0.4 mm. The sucked liquid was an eye perfusion liquid. The suction was performed by a venturi pump (an example of a suction device) and the suction amount per minute was measured while changing a suction pressure. The measurement result is shown in Table 1.

**[Table 1]**

| | | | | | | | | | | | | |
|---|---|---|---|---|---|---|---|---|---|---|---|---|
| Suction pressure (mmHg) | 50 | 100 | 150 | 200 | 250 | 300 | 350 | 400 | 450 | 500 | 550 | 600 |
| First inner through-hole (cc/min) | 2.0 | 8.0 | 10.0 | 14.0 | 16.0 | 18.0 | 19.0 | 20.0 | 21.0 | 22.0 | 24.0 | 26.0 |
| Second tough-hole inner (cc/min) | 2.0 | 10.0 | 14.0 | 16.0 | 20.0 | 24.0 | 26.0 | 28.0 | 31.0 | 34.0 | 36.0 | 38.0 |

### Reference Signs List

1A, 1B, 1C, 1D, 1E: I/A handpiece (surgical tool), 2: grip portion (holding portion), 3A, 3B, 3C, 3D, 3E: nozzle portion, 4: inner tube, 4b: side portion, 5: outer tube, 5b: side portion, 7: movable body, 8: casing portion, 12: handle portion, 13a: O-ring (ring member), 21: discharge side casing (first casing), 21a: discharge side concave portion (concave portion), 22: introduction side casing (second casing), 23a: rod main body, 23b: head portion, 23c: remaining portion, 23E: connection portion (guide rod), 41, 42, 44, 45, 46: inner through-hole, 47: first inner through-hole, 48: second inner through-hole, 51, 52, 53, 54: outer through-hole, 71: escaping hole (guide hole), Dr: rotation direction, 71a: first guide surface, 71b: second guide surface, 71c, 71d: stopper surface, K: movement locus, La: suction liquid, Sf: axis, Ra: inner passage, Rc: enlarged passage portion, Ly: length of enlarged passage portion, Lx: length of inner tube, Dx: distance from O-ring to end portion of discharge side concave portion, Dy: length of remaining portion, Wa: distance between first guide surface and second guide surface of first region, Wb: distance between first guide surface and second guide surface of second region, dx: maximum outer diameter of rod main body, dy: outer diameter of head portion.

## Claims

1. A surgical tool for ophthalmology that includes a nozzle portion capable of sucking a liquid,
wherein the nozzle portion includes an inner tube which is provided with an inner through-hole allowing the liquid to pass therethrough and an outer tube which accommodates the inner tube and is provided with an outer through-hole allowing the liquid to pass therethrough,
wherein the outer tube and the inner tube are disposed to be relatively movable, and
wherein the outer through-hole and the inner through-hole are disposed such that an overlapping region is changeable by the relative movement of the outer tube and the inner tube.

2. The surgical tool according to claim 1,
wherein the outer through-hole and the inner through-hole are disposed so that at least a part of one of the through-holes overlaps a movement locus of at least the other one that moves.

3. The surgical tool according to claim 1 or 2,
wherein the inner tube is movable with respect to the outer tube and at least a part of the outer through-hole is disposed to overlap a movement locus of the inner through-hole.

4. The surgical tool according to any one of claims 1 to 3,
wherein the outer through-hole is provided in a side portion of the outer tube and the inner through-hole is provided in a side portion of the inner tube.

5. The surgical tool according to any one of claims 1 to 4,
wherein the outer tube and the inner tube have a common axis, and
wherein the surgical tool further comprises a holding portion which holds the outer tube and the inner tube to be relatively movable in a rotation direction with respect to the axis.

6. The surgical tool according to any one of claims 1 to 5,
wherein the outer through-hole is larger than the inner through-hole, and
wherein the inner through-hole is housed in the outer through-hole in a state in which the outer through-hole and the inner through-hole completely overlap each other.

7. The surgical tool according to claim 6,
wherein the inner tube is provided with a plurality of the inner through-holes having different sizes.

8. The surgical tool according to any one of claims 1 to 7, further comprising:
a movable body which is fixed to the inner tube and is provided with an inner passage communicating with the inner tube; and
a casing portion which is fixed to the outer tube, accommodates the movable body, and rotatably supports the movable body,
wherein the movable body is provided with a handle portion which protrudes in a direction orthogonal to an axis and penetrates the casing portion to be exposed from the casing portion.

9. A surgical tool for ophthalmology comprising:
a nozzle portion which is able to suck a liquid; and
a holding portion which is fixed to the nozzle portion,
wherein the nozzle portion includes an inner tube through which a liquid passes and which is provided with a plurality of inner through-holes having different sizes and an outer tube which is provided with an outer through-hole allowing the liquid to flow therethrough, accommodates the inner tube, and has a common axis to the inner tube,
wherein the holding portion includes a movable body which is fixed to the inner tube and is provided with an inner passage communicating with the inner tube and a casing portion which is fixed to the outer tube, accommodates the movable body, and rotatably supports the movable body, and
wherein at least a part of the outer through-hole is disposed to overlap the on a movement locus of the plurality of inner through-holes.

10. The surgical tool according to claim 9,
wherein the inner passage includes an enlarged passage portion which has an inner diameter larger than that of the inner tube, and
wherein a length in a direction along the axis of the enlarged passage portion is longer than a length of the inner tube.

11. The surgical tool according to claim 9 or 10,
wherein the plurality of inner through-holes are a first inner through-hole and a second inner through-hole larger than the first inner through-hole,
wherein the movable body is provided with a handle portion which protrudes in a direction orthogonal to the axis and penetrates the casing portion to be exposed from the casing portion,
wherein the handle portion is provided with a guide hole which penetrates in a direction along the axis and is elongated along a rotation direction around the axis when viewed from a direction along the axis,
wherein the casing portion includes a guide rod which is inserted through the guide hole and is relatively movable inside the guide hole,
wherein the guide hole is formed by a first guide surface which extends along the rotation direction around the axis, a second guide surface which is away from the axis in relation to the first guide surface and extends to face the first guide surface, and a pair of stopper surfaces which is provided at both ends of the first guide surface and the second guide surface and comes into contact with the guide rod to regulate the relative movement of the guide rod, and
wherein the first inner through-hole and the outer through-hole overlap each other at a position in which the guide rod comes into contact with one stopper surface of the pair of stopper surfaces and the second inner through-hole and the outer through-hole overlap each other at a position in which the guide rod comes into contact with the other stopper surface.

12. The surgical tool according to claim 11,
wherein the first inner through-hole and the second inner through-hole are provided at a position in which a rotation angle around the axis is 180°.

13. The surgical tool according to claim 11 or 12,
wherein the casing portion and the movable body are separable from each other,
wherein the casing portion includes a first casing to which the outer tube is fixed and a second casing which is opposite to the first casing with the handle portion interposed therebetween, and
wherein the guide rod includes a rod main body which is fixed to the first casing and a head portion which is attached to an end of the rod main body and is separably attached to the second casing.

14. The surgical tool according to claim 13,
wherein the rod main body has a linear shape extending along the axis,
wherein an outer diameter of the head portion is larger than a maximum outer diameter of the rod main body,
wherein the guide hole includes a first region in which a distance between the first guide surface and the second guide surface is larger than the maximum outer diameter of the rod main body and smaller than the outer diameter of the head portion and a second region in which a distance between the first guide surface and the second guide surface is larger than the outer diameter of the head portion, and
wherein the second region is narrower than the first region in a rotation direction around the axis and extends along the axis.

15. The surgical tool according to claim 14,
wherein the movable body includes a protrusion shaft body which protrudes from the handle portion and is separably accommodated in the first casing along the axis and an annular seal member that is attached to the protrusion shaft body and is in contact with the first casing,
wherein the first casing includes a concave portion which accommodates the protrusion shaft body and is in contact with the seal member,
wherein the rod main body includes a remaining portion which forms a gap between the handle portion and the head portion, and
wherein a distance from the seal member to an end portion near the handle portion of the concave portion is equal to or longer than a length in a direction along the axis of the remaining portion.

16. The surgical tool according to claim 15,
wherein a distance from the seal member to an end portion near the handle portion of the concave portion is the same as a length in a direction along the axis of the remaining portion.
